(19) Europäisches Patentamt
European Patent Office
Office européen des brevets

(11) EP 0 983 235 B1

(12) **EUROPÄISCHE PATENTSCHRIFT**

(45) Veröffentlichungstag und Bekanntmachung des Hinweises auf die Patenterteilung:
**03.07.2002 Patentblatt 2002/27**

(21) Anmeldenummer: **98930743.4**

(22) Anmeldetag: **22.05.1998**

(51) Int Cl.7: **C07C 405/00**
// A61K31/557

(86) Internationale Anmeldenummer:
**PCT/EP98/03139**

(87) Internationale Veröffentlichungsnummer:
**WO 98/52915 (26.11.1998 Gazette 1998/47)**

(54) **LEUKOTRIEN-B4 DERIVATE, INSBESONDERE OXIMO-LTB4 ANTAGONISTEN**

LEUKOTRIENE B4 DERIVATIVES, IN PARTICULAR OXIMO-LTB4 ANTAGONISTS

DERIVES DE LEUCOTRIENE B4, EN PARTICULIER ANTAGONISTES D'OXIMO-LTB4

(84) Benannte Vertragsstaaten:
**AT BE CH CY DE DK ES FI FR GB GR IE IT LI LU MC NL PT SE**

(30) Priorität: **23.05.1997 DE 19722848**

(43) Veröffentlichungstag der Anmeldung:
**08.03.2000 Patentblatt 2000/10**

(73) Patentinhaber: **SCHERING AKTIENGESELLSCHAFT**
**13342 Berlin (DE)**

(72) Erfinder:
- **BUCHMANN, Bernd**
  **D-16540 Hohen Neuendorf (DE)**
- **FRÖHLICH, Wolfgang**
  **D-10709 Berlin (DE)**
- **GIESEN, Claudia**
  **D-13587 Berlin (DE)**
- **HENNEKES, Hartwig**
  **D-13507 Berlin (DE)**
- **JAROCH, Stefan**
  **D-10625 Berlin (DE)**
- **SKUBALLA, Werner**
  **D-13465 Berlin (DE)**

(74) Vertreter: **Dörries, Hans Ulrich, Dr. et al**
**Dörries, Frank-Molnia & Pohlman,**
**Triftstrasse 13**
**80538 München (DE)**

(56) Entgegenhaltungen:
**EP-A- 0 581 187** **WO-A-95/20563**

- **VLATTAS, ISIDOROS ET AL: "O-(1-Alkyl- or arylthioalkyl)hydroxylamines. New class of oxime reagents, their preparation, and synthetic utility" J. ORG. CHEM. (1973), 38(21), 3749-52 CODEN: JOCEAH,1973, XP002084528**
- **HAMANAKA, NOBUYUKI ET AL: "Molecular design of novel PGI2 agonists without PG skeleton. II" BIOORG. MED. CHEM. LETT. (1995), 5(10), 1071-6 CODEN: BMCLE8;ISSN: 0960-894X,1995, XP002084529**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft neue Leukotrien $B_4$-Derivate, Verfahren zu ihrer Herstellung sowie ihre Verwendung als Arzneimittel. Die neuen Verbindungen sind optisch aktive Strukturanaloga von vorbekannten Leukotrien-$B_4$-Antagonisten, die einen Sechsring als Grundstrukturelement enthalten (DE-A 39 17 597, DE-A 42 27 790.6, DE 42 42 390).

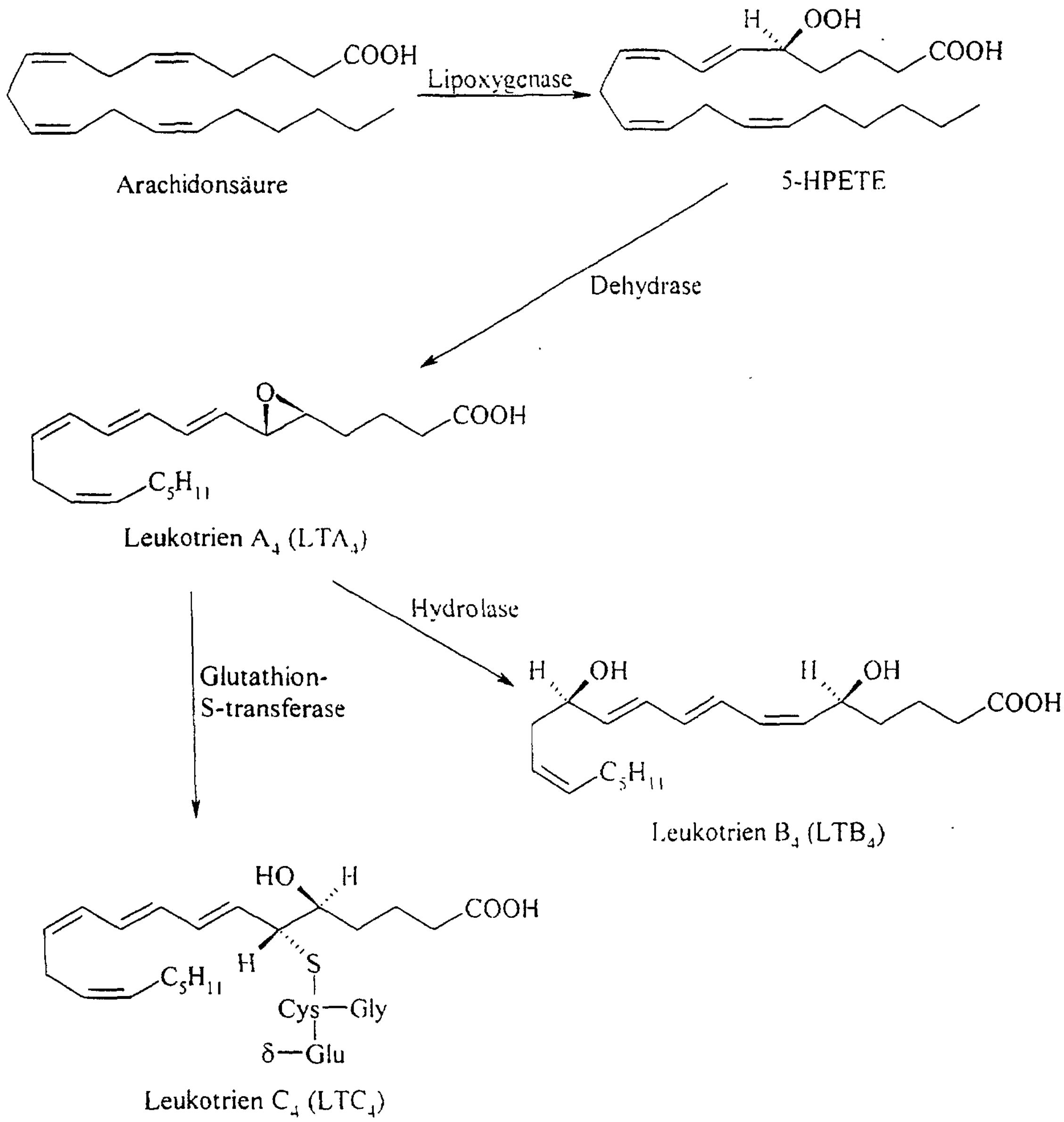


Arachidonsäure

5-HPETE

Leukotrien $A_4$ ($LTA_4$)

Leukotrien $B_4$ ($LTB_4$)

Leukotrien $C_4$ ($LTC_4$)

Leukotrien $B_4$ ($LTB_4$) wurde von B. Samuelsson und Mitarbeitern als Metabolit der Arachidonsäure entdeckt. Bei der Biosynthese wird durch das Enzym 5-Lipoxygenase zunächst als zentralens Zwischenprodukt das Leukotrien $A_4$ gebildet, das dann durch eine spezifische Hydrolase in das $LTB_4$ umgewandelt wird.

Die Nomenklatur der Leukotriene kann folgenden Arbeiten entnommen werden:

a) B. Samuelsson et al., Prostaglandins 19, 654 (1980); 17, 785 (1979).
b) C. N. Serhan et al., Prostaglandins 34, 201 (1987)

Die physiologische und insbesondere die pathophysiologische Bedeutung des Leukotrien $B_4$ wird in einigen neueren Arbeiten zusammengefaßt: a) The Leukotriens, Chemistry and Biology eds. L. W. Chakrin. D. M. Bailey. Academic Press 1984. b) J. W. Gillard et al.; Drugs of the Future 12. 453 (1987). c) B. Samuelsson., Sciences 237. 1171

(1987). d) C. W. Parker, Drug Development Research 10, 277 (1987). e) W. R. Henderson, Annals of Internal Medicine 121, 684 (1994). Hieraus ergibt sich, daß $LTB_4$ ein wichtiger Entzündungsmediator für entzündliche Erkrankungen ist, bei denen Leukozyten in das erkrankte Gewebe einwandern.
Die Effekte von $LTB_4$ werden auf zellulärer Ebene durch die Bindung von $LTB_4$ an einen spezifischen Rezeptor ausgelöst.
Von $LTB_4$ weiß man, daß es die Adhäsion von Leukozyten an die Blutgefäßwand verursacht $LTB_4$ ist chemotaktisch wirksam, d. h. es löst eine gerichtete Wanderung von Leukozyten in Richtung eines Gradienten steigender Konzentration aus. Außerdem verändert es aufgrund seiner chemotaktischen Aktivität indirekt die vasculäre Permeabilität, wobei ein Synergismus mit Prostaglandin $E_2$ beobachtet wird. $LTB_4$ spielt offensichtlich bei entzündlichen, allergischen und immunologischen Prozessen eine entscheidende Rolle.

Leukotriene und insbesondere $LTB_4$ sind an Hauterkrankungen beteiligt, die mit entzündlichen Prozessen (erhöhte Gefäßpermeabilität und Ödembildung, Zellinfiltration), erhöhter Proliferation der Hautzellen und Juckreiz einhergehen, wie beispielsweise bei Ekzemen, Erythemen, Psonasis, Pruritus und Akne. Pathologisch erhöhte Leukotrien-Konzentrationen sind an der Entstehung vieler Dermatitiden entweder ursächlich beteiligt, oder es besteht ein Zusammenhang zwischen der Persistenz der Dermatitiden und den Leukotrienen. Deutlich erhöhte Leukotrienkonzentrationen wurden beispielsweise in der Haut von Patienten mit Psoriasis, atopischer Dermatitis, allergischer Kontaktdermatitis, bullöse Pemphigoide, verzögerte Duchurtikaria und allergischer Vasculitis gemessen.

Leukotriene und insbesondere $LTB_4$ sind auch an Erkrankungen innerer Organe beteiligt, für die eine akute oder chronische entzündliche Komponente beschrieben wurde, z. B.: Gelenkerkrankungen (Rheumatische Arthritis); Erkrankungen des Respirationstraktes (Asthma und chronisch obstruktive Lungenerkrankungen (OPD)); entzündliche Darmerkrankungen (ulceröse Colitis und Morbus Crohn); sowie Reperfusionsschäden (an Herz-, Darm- oder Nierengewebe), die durch zeitweisen krankhaften Verschluß von Blutgefäßen entstehen. wie Glomerulonephritis, NSAID Gastropathien, Multiple Sklerose, Rhinitis und entzündliche Augenerkrankungen. Weiterhin sind Leukotriene und insbesondere $LTB_4$ bei der Erkrankung an multipler Sklerose beteiligt und bei dem klinischen Erscheinungsbild des Schocks (ausgelöst durch Infektionen, Verbrennungen oder bei Komplikationen bei der Nierendialyse oder anderen extra besprochenen Perfusionstechniken).

Leukotriene und insbesondere $LTB_4$ haben weiterhin einen Einfluß auf die Bildung von weißen Blutkörperchen im Knochenmark, auf das Wachstum von glatten Muskelzellen, von Keratinozyten und von B-Lymphozyten. $LTB_4$ ist daher bei Erkrankungen mit entzündlichen Prozessen und bei Erkrankungen mit pathologisch gesteigerter Bildung und Wachstum von Zellen beteiligt.
Erkrankungen mit diesem Erscheinungsbild stellen z. B. Leukemie oder Artherosklerose dar.

Leukotriene und insbesondere $LTB_4$ und seine Derivate sind zur Senkung erhöhter Triglyceridspiegel geeignet und wirken somit antiatherosklerotisch und gegen Fettleibigkeit.

Durch die Antagonisierung der Effekte insbesondere von $LTB_4$ sind die Wirkstoffe und deren Darreichungsformen dieser Erfindung spezifische Heilmittel bei der Erkrankung von Mensch und Tier, bei denen insbesondere Leukotriene eine pathologische Rolle spielen.

Neben therapeutischen Möglichkeiten, die sich aus einer Antagonisierung der $LTB_4$-Wirkung mit $LTB_4$-Analoga ableiten lassen, konnte auch die Nützlichkeit und potentielle Anwendung von Leukotrien $B_4$-Agonisten zur Behandlung von Pilzerkrankungen der Haut gezeigt werden (H. Katayama. Prostaglandins 34, 797 (1988)).

Die Erfindung betrifft Leukotrien-$B_4$-Derivate der allgemeinen Formel I,

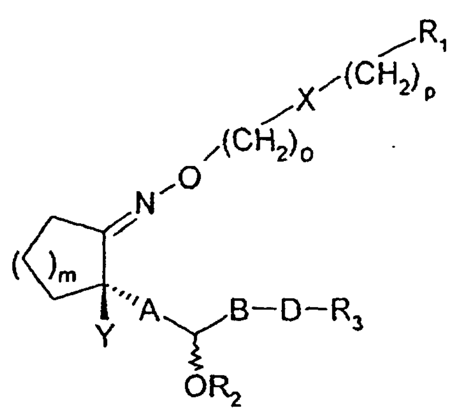

(I)

worin

$R_1$ H, $CF_3$, $CH_2OH$, $COOR_4$, $CONR_5R_6$ und
$R_2$ H oder einen organischen Säurerest mit 1-15 C-Atomen darstellt,
$R_3$ H, gegebenenfalls einfach oder mehrfach substituiertes $C_1$-$C_{14}$-Alkyl, $C_3$-$C_{10}$-Cycloalkyl, einen gegebenenfalls unabhängig voneinander einfach oder mehrfach durch
Halogen, Phenyl, $C_1$-$C_4$-Alkyl, $C_1$-$C_4$-Alkoxy, Fluormethyl, Chlormethyl, Trifluormethyl, Carbonyl, Carboxyl oder Hydroxyl substituierten $C_6$-$C_{10}$-Arylrest oder einen 5-6 gliedrigen aromatischen heterocyclischen Ring mit wenigstens 1 Heteroatom symbolisiert.
$R_4$ Wasserstoff, $C_1$-$C_{10}$-Alkyl, $C_3$-$C_{10}$-Cycloalkyl, einen gegebenenfalls durch 1-3 Halogen,
Phenyl, $C_1$-$C_4$-Alkyl, $C_1$-$C_4$-Alkoxy, Fluormethyl, Chlormethyl, Trifluormethyl, Carboxyl oder Hydroxyl substituierten $C_6$-$C_{10}$-Arylrest, $CH_2$-CO-($C_6$-$C_{10}$) Aryl oder einen 5-6 gliedrigen Ring mit wenigstens 1 Heteroatom bedeutet.
A eine trans, trans-CH=CH-CH=CH, eine -$CH_2CH_2$-CH=CH- oder eine Tetramethylengruppe,
B eine $C_1$-$C_{10}$- gerad- oder verzweigtkettige Alkylengruppe, die gegebenenfalls durch Fluor substitiuert sein kann oder die Gruppe

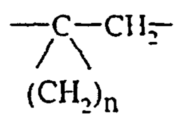 oder 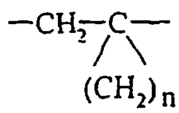

symbolisiert,
D eine Direktbindung, Sauerstoff, Schwefel, -C≡C-, -CH=$CR_7$, oder gemeinsam mit
B auch eine Direktbindung bedeuten kann.
$R_5$ und $R_6$ gleich oder verschieden sind und H oder gegebenenfalls durch Hydroxygruppen substituiertes $C_1$-$C_4$-Alkyl oder $R_6$ H und $R_5$ $C_1$-$C_{15}$-Alkanoyl oder $R_8SO_2$- darstellen,
$R_7$ H, $C_1$-$C_5$-Alkyl, Chlor, Brom bedeutet,
$R_8$ die gleiche Bedeutung wie $R_3$ besitzt,
m 1-3 bedeutet
o 0-5 bedeutet,
p 0-4
X eine Direktbindung, Sauerstoff, Schwefel, einen Aromaten oder Heteroaromaten,
Y ein gegebenenfalls einfach oder mehrfach substituiertes $C_1$-$C_8$-Alkyl, $C_3$-$C_{10}$-Cycloalkyl, gegebenenfalls substituiert durch Aryl,
n 2-5 ist

und, wenn $R_4$ Wasserstoff bedeutet, deren Salze mit physiologisch verträglichen Basen und deren Cyclodextrinclathrate.

Die Gruppe $OR_2$ kann α- oder β-ständig sein. Die Formel I umfaßt sowohl Racemate als auch die möglichen reinen Diastereomeren und Enantiomeren. Die Stereochemie der Oximdoppelbindung kann *E*- oder *Z*-konfigunert sein; vorzugsweise werden *E*-konfigurierte Oxime erhalten.

Als Alkylgruppen $R_4$ kommen gerad- oder verzweigtkettige Alkylgruppen mit 1-10 C-Atomen in Betracht, wie beispielsweise Methyl, Ethyl, Propyl, Butyl, Isobutyl, tert.-Butyl, Pentyl, Neopentyl, Hexyl, Heptyl, Decyl.
Die Alkylgruppen $R_4$ können gegebenenfalls ein- bis mehrfach substituiert sein durch Halogenatome, Alkoxygruppen, gegebenenfalls substituierte Aryl- bzw. Aroylgruppen mit 6-10 C-Atomen (bezüglich möglicher Substituenten siehe unter Aryl $R_4$), Dialkylamino und Trialkylammonium mit 1-4 C-Atomen im Alkyl-Teil, wobei die einfache Substitution bevorzugt sein soll. Als Substituenten seien beispielsweise genannt Fluor, Chlor oder Brom, Phenyl, Dimethylamino, Diethylamino, Methoxy, Ethoxy. Als bevorzugte Alkylgruppen $R_4$ sind solche mit 1-4 C-Atomen zu nennen.

Die Cycloalkylgruppe $R_4$ kann im Ring 3-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl, Cyclohexyl, Methylcyclohexyl.

Als Arylgruppen $R_4$ kommen sowohl substituierte als auch unsubstituierte Arylgruppen mit 6-10 C-Atomen in Betracht, wie beispielsweise Phenyl, 1-Naphthyl und 2-Naphthyl, die jeweils substiuiert sein können duch 1-3 Halogenatome (F, Cl, Br), eine Phenylgruppe, 1-3 Alkylgruppen mit jeweils 1-4 C-Atomen, eine Chlormethyl-, eine Fluormethyl-, Trifluormethyl-, Carboxyl-, Hydroxyl- oder Alkoxygruppe mit 1-4 C-Atomen. Bevorzugte Substituenten in 3- und 4-Stellung am Phenylring sind zum Beispiel Fluor, Chlor, Alkoxy oder Trifluormethyl, in 4-Stellung dagegen Hy-

droxyl.

Als heterocyclische Gruppen $R_4$ kommen 5- und 6-gliedrige aromatische Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, 3-Furyl, 3-Thienyl, 2-Tetrazolyl u. a.

Als Säurerest $R_5$ kommen solche physiologisch verträglichen Säuren in Frage. Bevorzugte Säuren sind organische Carbonsäuren und Sulfonsäuren mit 1-15 Kohlenstoffatomen, die der aliphatischen, cycloaliphatischen, aromatischen, aromatisch-aliphatischen und heterocyclischen Reihe angehören. Diese Säuren können gesättigt, ungesättigt und/oder mehrbasisch und/oder in üblicher Weise substituiert sein. Als Beispiele für die Substituenten seien $C_{1-4}$-Alkyl-, Hydroxyl-, $C_{1-4}$-Alkoxy-, Oxo- oder Aminogruppen oder Halogenatome (F, Cl, Br) erwähnt. Beispielweise seien folgende Carbonsäuren genannt: Ameisensäure. Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyessigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen (F, Cl, Br) oder Trifluormethyl-, Hydroxy, $C_{1-4}$-Alkoxy- oder Carboxy-Gruppen substituierte Benzoesäuren. Nikotinsäure, Isonikotinsäure, Furan-2-carbonsäure, Cyclopentylpropionsäure. Als bevorzugte Arylsulfonylreste und Alkansulfonylreste $R_8SO_2$- werden solche betrachtet, die sich von einer Sulfonsäure, mit bis zu 10 Kohlenstoffatomen ableiten. Als Sulfonsäuren kommen beispielsweise Methansulfonsäure, Ethansulfonsäure, Isopropansulfonsäure, Cyclohexansulfonsäure, Benzolsulfonsäure, p-Toluolsulfonsäure, p-Chlorbenzolsufonsäure, N,N-Dimethylaminosulfonsäure, N,N-Diisobutylaminosufonsäure, N,N-Dibutylaminosulfonsäure, Pyrrolidino-, Piperidino-, Piperazino-, M-Methylpiperazino- und Morpholinosulfonsäure in Frage.

Als Alkylgruppen $R_3$ kommen gerad- und verzweigtkettige, gesättigte und ungesättigte Alkylreste, vorzugsweise gesättigte, mit 1-14, insbesondere 1-10 C-Atomen, in Frage, die gegebenenfalls durch gegebenenfalls substituiertes Phenyl (Substitution s. unter Aryl $R_5$) substituiert sein können. Beispielsweise seien genannt Methyl-, Ethyl-, Propyl-, Butyl-, Isobutyl-, tert.-Butyl-, Pentyl-, Hexyl-. Heptyl-, Octyl-, Butenyl-, Isobutenyl-, Propenyl-, Pentenyl-, Benzyl-, m- und p-Chlorbenzylgruppen. Sind die Alkylgruppen $R_3$ Halogen-substituiert, kommen als Halogene Fluor, Chlor und Brom in Frage.

Als Beispiele für Halogen-substituierte Alkylgruppen $R_3$ kommen Alkyle mit terminalen Tri-fluormethylgruppen in Betracht.

Die Cycloalkylgruppe $R_3$ kann im Ring 3-10, vorzugsweise 3-6 Kohlenstoffatome enthalten. Die Ringe können durch Alkylgruppen mit 1-4 Kohlenstoffatomen gegebenenfalls durch Halogene substituiert sein. Beispielsweise genannt seien Cyclopropyl, Cyclobutyl, Cyclopentyl, Cyclohexyl, Methyl-cyclohexyl, Fluor-cyclohexyl.

Als substituierte bzw. unsubstitiuerte Arylgruppen $R_3$ kommen beispielweise in Betracht: Phenyl, 1-Naphthyl und 2-Naphthyl, die jeweils substituiert sein können durch 1-3 Halogenatome (F, Cl. Br), eine Phenylgruppe, 1-3 Alkylgruppen mit jeweils 1-4 C-Atomen, eine Chlormethyl-, Fluormethyl-, Trifluormethyl-, Carboxyl-, $C_1$-$C_4$-Alkoxy- oder Hydroxylgruppe. Bevorzugt ist die Substitution in 3- und 4-Stellung am Phenylring zum Beispiel durch Fluor, Chlor, Alkoxy oder Trifluormethyl oder in 4-Stellung durch Hydroxyl.

Als heterocyclische aromatische Gruppen $R_3$ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 1-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, u. a.

Als Alkylengruppen B kommen geragkettige oder verzweigte, gesättigte oder ungesättigte Alkylenreste, vorzugsweise gesättigte mit 1-10, insbesondere mit 1-5 C-Atomen, in Frage, die gegebenenfalls durch Fluoratome substituiert sein können. Beispielsweise seien genannt: Methylen, Fluormethylen, Difluormethylen, Ethylen, 1,2-Propylen, Ethylethylen, Trimethylen, Tetramethylen, Pentamethylen, 1,2-Difluorethylen, 1-Fluorethylen, 1-Methyltetramethylen, 1-Methyl-trimethylen, 1-Methylen-ethylen, 1-Methylen-tetramethylen.

Die Alkylengruppe B kann weiterhin die Gruppe

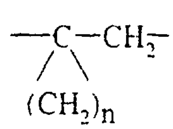

oder

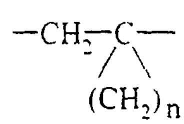

darstellen, wobei n=2-5, bevorzugt 3-5 bedeutet.

Als Säurereste $R_2$ kommen solche von physiologisch verträglichen Säureresten in Frage. Bevorzugte Säuren sind organische Carbonsäuren und Sulfonsäuren mit 1-15 Kohlenstoffatomen, die der aliphatischen, cyclo-aliphatischen, aromatischen, aromatisch-aliphatischen oder heterocyclischen Reihe angehören. Diese Säuren können gesättigt, ungesättigt und/ oder mehrbasisch und/ oder in üblicher Weise susbtituiert sein. Als Beispiele für die Substituenten seien $C_{1-4}$-Alkyl-, Hydroxyl-, $C_{1-4}$-Alkoxy-, Oxo- oder Aminogruppen oder Halogenatome (F, Cl, Br) erwähnt. Beispielsweise seien folgende Carbonsäuren genannt: Ameisensäure. Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, Cyclopentylessigsäure, Cyclohexylessigsäure, Cyclohexancarbonsäure, Phenylessigsäure, Phenoxyessigsaure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen (F, Cl, Br) oder Trifluormethyl-, Hydroxyl-, $C_{1-4}$-Alkoxy- oder Carboxy-Gruppen substituierte Benzoesäuren, Nikotinsäure, Isonicotinsäure, Furan-2-carbonsäure, Cyclopentylpropionsäure. Als bevorzugte Säurereste $R_2$ und $R_3$ werden solche Acylreste mit bis zu 10 Kohlenstoffatomen betrachtet.

Die Alkylreste $R_5$ und $R_6$, die gegebenenfalls Hydroxygruppen enthalten, sind geradkettige oder verzweigte Alkylreste, insbesondere geradkettige wie zum Beispiel Methyl, Ethyl, Propyl, Butyl, Pentyl, Hexyl, besonders bevorzugt Methyl.

$R_7$ als $C_{1-5}$-Alkyl bedeutet geradkettige oder verzweigtkettige Alkylreste wie sie für $R_3$ bzw. $R_4$ bereits genannt wurden. Bevorzugte Alkylreste $R_7$ sind Methyl, Ethyl, Propyl und Isopropyl.

Zur Salzbildung sind anorganische und organische Basen geeignet, wie sie dem Fachmann zur Bildung physiologisch verträglicher Salze bekannt sind. Beispielsweise seien genannt Alkalihydroxide wie Natrium- und Kaliumhydroxid, Erdalkalihydroxide wie Calciumhydroxid, Ammoniak, Amine, wie Ethanolamin, Diethanolamin, Triethanolamin, N-Methylglucamin, Morpholin, Tris-(hydroxymethyl)-methylamin usw.

Um zu den Cyclodextrinclathraten zu gelangen werden die Verbindungen der Formel I mit α-, β- oder γ-Cyclodextrin umgesetzt. Bevorzugt sind β-Cyclodextrinderivate.

Bevorzugte Verbindungen der vorliegenden Erfindung sind Verbindungen der allgemeinen Formel I, wobei die Reste die folgende Bedeutung haben:

| | |
|---|---|
| $R_1$ | ist $CF_3$, $CH_2OH$, $CONR_5R_6$, $COOR_4$ mit $R_4$ in der Bedeutung eines Wasserstoffatoms, eines Alkylrestes mit 1-10 C-Atomen, eines Cycloalkylrestes mit 5-6 C-Atomen, eines gegebenenfalls durch 1-2 Chlor, Brom, Phenyl, $C_{1-4}$-Alkyl, $C_{1-4}$- Alkoxy, Chlormethyl, Fluormethyl, Trifluormethyl, Carboxy oder Hydroxy substituierten Phenylrestes. |
| X | ist ein Aromat oder eine Direktbindung |
| Y | ist eine Methylgruppe |
| m | ist 1-3 |
| o | ist 1-5 |
| p | ist 0, 1 |
| A | ist eine trans-CH=CH-CH=CH- oder Tetramethylengruppe: |
| B | ist eine geradkettige oder verzweigtkettige gesättigte oder ungesättigte Alkylengruppe mit bis zu 10 C-Atomen, die gegebenenfalls durch Fluor substituiert sein kann, oder die Gruppe |

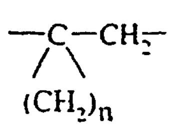

oder

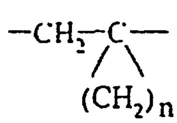

mit n=2-5;

| | |
|---|---|
| D | ist eine Direktbindung, Sauerstoff, Schwefel, eine -C≡C-Gruppe oder eine -CH=$CR_7$-Gruppe mit $R_7$ als Wasserstoff, $C_{1-5}$-Alkyl, Chlor oder Brom; |
| B und D | sind gemeinsam eine Direktbindung; |
| $R_2$ | bedeutet Wasserstoff oder einen organischen Säurerest mit 1-15 C-Atomen; |
| $R_5$ und $R_6$ | weisen die oben angegebenen Bedeutungen auf; |
| $R_3$ | ist ein Wasserstoffatom, $C_{1-10}$-Alkyl, Cycloalkyl mit 5-6 C-Atomen, ein gegebenenfalls durch 1-2 Chlor, Brom, Phenyl, $C_{1-4}$-Alkyl, $C_{1-4}$-Alkoxy, Chlormethyl, Fluormethyl, Trifluormethyl, Carboxyl oder Hydroxyl substituierter Phenylrest und, falls |

$R_4$ ein Wasserstoff bedeutet, deren Salze mit physiologisch verträglichen Basen und Cyclodextrinclathrate.

Besonders bevorzugte Verbindungen der vorliegenden Erfindung sind Verbindungen der allgemeinen Formel I, wobei die Reste folgende Bedeutung haben:

- $R_1$ ist $CF_3$, $CH_2OH$, $CONR_5R_6$, $COOR_4$ mit $R_4$ in der Bedeutung eines Wasserstoffatoms, eines Alkylrestes mit 1-4 C-Atomen
- $R_2$ bedeutet Wasserstoff oder einen organischen Säurerest mit 1-6 C-Atomen:
- $R_3$ ist ein Wasserstoffatom oder $C_{1-10}$-Alkyl:
- $R_5$ und $R_6$ weisen die oben angegebenen Bedeutungen auf;
- A ist eine trans, trans-CH=CH-CH=CH- oder Tetramethylengruppe,
- B ist eine geradkettige oder verzweigtkettige Alkylengruppe mit bis zu 5 C-Atomen oder die Gruppe

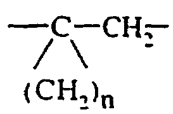 oder 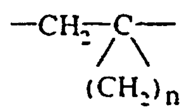

mit n=3,4

- D ist eine Direktbindung oder eine -C≡C-Gruppe oder eine $-CH=CR_7$-Gruppe mit $R_7$ als Wasserstoff oder $C_{1-5}$-Alkyl;
- X ist eine Direktbindung oder ein Aromat
- Y ist eine Methylgruppe
- m ist 2
- o ist 1-5
- p ist 0, 1
- B und D sind gemeinsam eine Direklbindung;

und falls $R_4$ ein Wasserstoffatom bedeutet, deren Salze mit physiologisch verträglichen Basen und deren Cyclodextrinclathrate.

Außerdem betrifft die Erfindung ein Verfahren zur Herstellung der erfindungsgemäßen Verbindungen der allgemeinen Formel I, das dadurch gekennzeichnet ist, daß man ein Keton der Formel II,

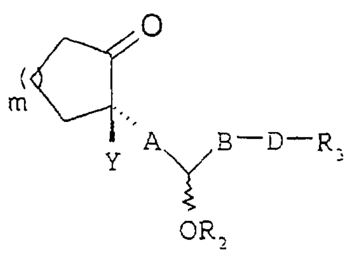

(II)

worin A, B, D, m, $R_2$, $R_3$ und Y die oben angegebene Bedeutung haben, gegebenenfalls nach Schutz freier Hydroxygruppen in $R_2$ mit einem Hydroxylamin oder einem Hydroxylammoniumsalz umsetzt und anschließend mit einem Alkylierungsreagenz der allgemeinen Formel III.

$$E\text{-}(CH_2)_o\text{-}X\text{-}(CH_2)_p\text{-}R_1 \quad \text{(III)}$$

wobei E ein Halogenid oder Sulfonat darstellt und o, X, p, $R_1$ die oben angegebene Bedeutung aufweisen, in Gegenwart einer Base umsetzt und gegebenenfalls anschließend in beliebiger Reihenfolge Isomere trennt, geschützte Hydroxygruppen freisetzt und/ oder eine freie Hydroxygruppe verethert und/ oder eine Carboxylgruppe reduziert und/ oder eine Carboxylgruppe verestert und/ oder eine Carboxylgruppe in ein Amid überführt oder eine Carboxylgruppe mit einer physiologisch verträglichen Base in ein Salz überführt. Als Halogenide gemäß der allgemeinen Formel III kommen

Chlor, Brom, Iod, als Sulfonate Mesylat, Tosylat, Triflat infrage.

Die Umsetzung der Verbindung der allgemeinen Formel II mit einem Hydroxylammonium-Salz wird bei Temperaturen von 0°C bis 100°C, vorzugsweise bei 25°C in einem Lösungsmittelgemisch aus einem aprotischen Lösungsmittel, wie z.B. Tetrahydrofuran oder Pyridin, und einem protischen Lösungsmittel oder Lösungsmittelgemisch, wie Wasser und/ oder Alkoholen, vorgenommen.

Die Veretherung der Oxime mit Alkylierungsmitteln der allgemeinen Formel III erfolgt in bekannter Weise in einem aprotischen Lösungsmittel oder Lösungsmittelgemisch, beispeilsweise Dimethylformamid oder Tetrahydrofuran oder Dimethoxyethan, unter Einwirkung einer Base bei 0-30°C. Als Base kommt zum Beispiel Natriumhydrid infrage.

Die Veresterung der Alkohole der Formel I ($R_2$=H) erfolgt in an sich bekannter Weise. Zum Beispiel erfolgt die Veresterung dadurch, daß man ein Säurederivat, vorzugsweise ein Säurehalogenid oder Säureanhydrid, in Gegenwart einer Base wie beispielsweise Natriumhydrid, Pyridin, Triethylamin, Tributylamin oder 4-Dimethylaminopyridin mit einem Alkohol der Formel I umsetzt. Die Umsetzung kann ohne Lösungsmittel oder in einem inerten Lösungsmittel, vorzugsweise Aceton, Acetonitril, Dimethylacetamid, Dimethylsulfoxid bei Temperaturen über oder unter Raumtemperatur, zum Beispiel zwischen -80°C bis 100°C, vorzugsweise bei Raumtemperatur, vorgenommen werden.

Die Reduktion zu den Verbindungen der Formel I mit $R_1 = CH_2OH$ wird mit einem für die Reduktion von Estern oder Carbonsäuren geeigneten Reduktionsmittel wie beispielsweise Lithiumaluminiumhydrid, Diisobutylaluminumhydrid usw. durchgeführt. Als Lösungsmittel kommen Diethylether, Tetrahydrofuran, Dimethoxyethan, Toluol usw. infrage. Die Reduktion wird bei Temperaturen von -30 °C bis zur Siedetemperatur des Lösungsmittels, vorzugsweise von 0 - 30 °C vorgenommen

Die Veretherung der Alkohole der Formel I (mit $R_1$=$CH_2OH$ und p=0 und X eine Direktbindung) zu Verbindungen der allgemeinen Formel I (mit X=O und p=1-4) erfolgt in an sich bekannter Weise. Zum Beispiel erfolgt die Veretherung dadurch, daß man den Alkohol der allgemeinen Formel I ($R_1$=$CH_2OH$), gegebenenfalls nach Schutz anwesender freier Hydroxygruppen mit einem Halogencarbonsäurederivat oder Halogenalkylderivat der allgemeinen Formel IV.

$$\text{Hal-}(CH_2)_p\text{-}R_1 \qquad \text{(IV)}$$

wobei Hal ein Chlor-, Brom- oder Iodatom und $R_1$ die oben angegebene Bedeutung aufweist in Gegenwart einer Base umsetzt und anschließend gegebenenfalls $R_1$ wie oben beschrieben weiter funktionalisiert. Die Umsetzung der Verbindung der allgemeinen Formel I mit einer Halogenverbindung der allgemeinen Formel IV wird bei Temperaturen von 0°C bis 100°C, vorzugsweise 10°C bis 80°C, in einem aprotischen Lösungsmittel oder Lösungsmittelgemisch. beispielsweise Dimethylsulfoxid, Dimethylformamid, Tetrahydrofuran, Toluol usw.. vorgenommen. Als Basen kommen die dem Fachmann für Veretherungen bekannten Basen in Frage, beispielsweise Natriumhydrid, Kalium-tert.-butylat, Butyllithium. Die oben genannte Veretherung kann auch vorzugsweise unter Phasentransfer-Bedingungen mit 20-50%iger wäßriger Natriumhydroxid- oder Kaliumhydroxid Lösung ohne ein zusätzliches Lösungsmittel oder in einem aprotischen Lösungsmittel wie beispielsweise Toluol in Gegenwart eines Phasentransfer-Katalysators wie Tetrabutylammoniumhydrogensulfat bei Temperaturen zwischen 0°C und 90°C, vorzugsweise zwischen 20°C und 60°C durchgeführt werden.

Die Verseifung der Ester der Formel I wird nach den dem Fachmann bekannten Methoden durchgeführt, wie beispielsweise mit basischen Katalysatoren. Die Verbindungen der Formel I können durch die üblichen Trennmethoden in die optischen Isomeren getrennt werden (Asymmetric Synthesis, Vol. 1-5, Ed. J. D. Morrison, Academic Press. Inc. Orlando etc.. 1985; Chiral Separations by HPLC, Ed. A. M. Krstulovic; John Wiley & Sons; New York etc. 1989).

Die Freisetzung der geschützten Hydroxylgruppen erfolgt nach bekannten Methoden. Beispielsweise wird die Abspaltung von Hydroxylschutzgruppen, wie beispielsweise des Tetrahydropyranylrestes, in einer wäßrigen Lösung einer organischen Säure. wie z. B. Oxalsäure, Essigsäure, Propionsäure u. a., oder in einer wäßrigen Lösung einer Mineralsäure, wie z. B. Salzsäure durchgeführt. Zur Verbesserung der Löslichkeit wird zweckmäßigerweise ein mit Wasser mischbares inertes organisches Lösungsmittel zugesetzt. Geeignete organische Lösungsmittel sind z. B Alkohole, wie Methanol, Ethanol und Ether, wie Dimethoxyethan, Dioxan und Tetrahydrofuran, Tetrahydrofuran wird bevorzugt verwendet. Die Abspaltung wird vorzugsweise bei Temperaturen zwischen 20°C und 80°C durchgeführt. Die Abspaltung der Silyletherschutzgruppen erfolgt beispielsweise mit Tetrabutylammoniumfluorid oder mit Kaliumfluorid in Gegenwart eines Kronenethers (wie zum Beispiel Dibenzo[18]-Krone-6). Als Lösungsmittel sind beispielsweise geeignet Tetrahydrofuran, Diethylether, Dioxan, Dichlormethan, usw. Die Abspaltung wird vorzugsweise bei Temperaturen zwischen 0 °C und 80 °C durchgeführt.

Die Verseifung der Acylgruppen erfolgt beispielsweise mit Alkali- oder Erdalkalicarbonaten oder -hydroxiden in einem Alkohol oder in der wäßrigen Lösung eines Alkohols. Als Alkohol kommen niedere aliphatische Alkohole in Betracht, wie z. B. Methanol, Ethanol, Butanol usw.. vorzugsweise Methanol. Als Alkalicarbonate und - hydroxide seien

Kalium-, Natrium- und Caesiumsalze genannt. Bevorzugt sind Kaliumsalze.
Als Erdalkalicarbonate und -hydroxide sind beispielsweise geeignet Calciumcarbonat, Calciumhydroxid und Bariumcarbonat. Die Umsetzung erfolgt bei -10°C bis +70°C, vorzugsweise bei +25°C.
Die Einführung der Estergruppe $-COOR_4$ für $R_1$, bei welcher $R_4$ eine Alkylgruppe mit 1-10 C-Atomen darstellt, erfolgt nach den dem Fachmann bekannten Methoden. Die 1-Carboxy-verbindungen werden beispielsweise mit Diazokohlenwasserstoffen in an sich bekannter Weise umgesetzt. Die Veresterung mit Diazokohlenwasserstoffen erfolgt z. B. dadurch, daß man eine Lösung des Diazokohlenwasserstoffs in einem inerten Lösungsmittel, vorzugsweise in Diethylether, mit der 1-Carboxyverbindung in dem gleichen oder in einem anderen inerten Lösungsmittel, wie z. B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden (Org. Reactions Bd. 8, Seiten 389-394 (1954)).

Die Einführung der Estergruppe $-COOR_4$ für $R_1$, bei welcher $R_4$ eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base, beispielsweise Pyridin, Dimethylaminopyridin, Triethylamin, in einem inerten Lösungsmittel umgesetzt. Als Lösungsmittel kommen Methylenchlorid, Ethylenchlorid, Chloroform, Essigester, Tetrahydrofuran, vorzugsweise Chloroform in Frage. Die Reaktion wird bei Temperaturen zwischen -30°C und +50°C, vorzugsweise bei 10°C durchgeführt.

Die Leukotrien-$B_4$-Derivate der Formel I mit $R_4$ in der Bedeutung eines Wasserstoffs können mit geeigneten Mengen der entsprechenden anorganischen Basen unter Neutralisierung in ein Salz übergeführt werden Beispielsweise erhält man beim Lösen der entsprechenden Säuren in Wasser, das die stöchiometrische Menge der Base enthält, nach Abdampfen des Wassers oder nach Zugaben eines mit Wasser mischbaren Lösungsmittels, z. B. Alkohol oder Aceton, das (feste anorganische) Salz.

Zur Herstellung eines Ammoniumsalzes wird die freie Säure in einem geeigneten Lösungsmittel, beispielsweise Ethanol, Aceton, Diethylether, Acetonitril oder Benzol gelöst und mindestens die stöchiometrische Menge des Amins der Lösung zugesetzt. Dabei fällt das Salz gewöhnlich in fester Form an oder wird nach Verdampfen des Lösungsmittels in üblicher Weise isoliert.

Die Einführung der Amidgruppe-$CONHR_5$ mit $R_5$ in der Bedeutung von Alkanoyl erfolgt nach den dem Fachmann bekannten Methoden. Die Carbonsäuren der Formel I ($R_4$=H), werden zunächst in Gegenwart eines tertiären Amins, wie beispielsweise Triethylamin, mit Chlorameisensäure-isobutylester in das gemischte Anhydrid übergeführt. Die Umsetzung des gemischten Anhydrids mit dem Alkalisalz des entsprechenden Amids oder mit Ammoniak ($R_5$=H) erfolgt in einem inerten Lösungsmittel oder Lösungsmittelgemisch, wie beispielsweise Tetrahydrofuran, Dimethoxyethan, Dimethylformamid, Hexamethylphosphorsäuretriamid, bei Temperaturen zwischen -30°C und +60°C, vorzugsweise bei 0°C bis 30°C. Eine weitere Herstellungsart für die Amide besteht in der Amidolyse des 1-Esters ($R_1$=$COOR_4$) mit dem entsprechenden Amin.

Eine weitere Möglichkeit für die Einführung der Amidgruppe $-CONHR_5$ besteht in der Umsetzung einer 1-Carbonsäure der Formel I ($R_4$=H), in der freie Hydroxygruppen gegebenenfalls intermediär geschützt sind, mit Verbindungen der Formel IV,

$$O = C = N - R_5 \qquad (IV)$$

worin $R_5$ die oben angegebene Bedeutung hat.

Die Umsetzung der Verbindung der Formel I (R4 = H) mit einem Isocyanat der Formel IV erfolgt gegebenenfalls unter Zusatz eines tertiären Amins, wie z. B. Triethylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diethylether, Toluol, bei Temperaturen zwischen -80°C bis 100°C, vorzugsweise bei 0°C bis 30°C, vorgenommen werden.

Zur Herstellung der übrigen Amide kann man beispielsweise die gewünschten Säureanhydride mit Ammoniak oder den entsprechenden Aminen umsetzen.

Enthält das Ausgangsprodukt OH-Gruppen, so werden diese OH-Gruppen auch zur Reaktion gebracht. Werden letztlich Endprodukte gewünscht, die freie Hydroxylgruppen enthalten, geht man zweckmäßigerweise von Ausgangsprodukten aus, in denen diese durch vorzugsweise leicht abspaltbare Ether- oder Acylreste intermediär geschützt sind.

Die Trennung von Diastereomeren erfolgt nach den dem Fachmann bekannten Methoden, beispielsweise durch Säulenchromatographie.

Die als Ausgangsmaterial dienenden Verbindungen der allgemeinen Formel II können beispielsweise hergestellt werden, in dem man in an sich bekannter Weise einen Ester der allgemeinen Formel V((a) K. Sakai et al.. Tetrahedron 50, 3315 (1995); b) K. Koga et al., Tetrahedron 49, 1579 (1993)).

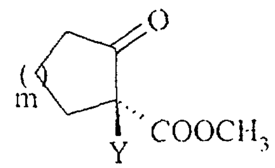

(V)

worin m und Y die oben angegebenen Bedeutungen aufweisen, mit Ethylenglycol ketalisiert, mit Diisobutylaluminiumhydrid reduziert und anschließend mit dem Collins-Reagenz oder durch das Swern-Verfahren (Tetrahedron Letters 34, 1651 (1978)) zum Aldehyd der allgemeinen Formel VI oxidiert.

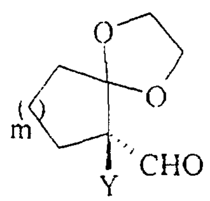

(VI)

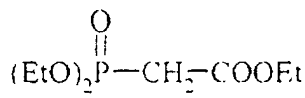

(VII)

oder

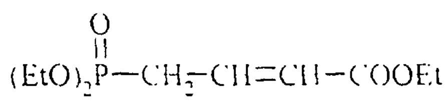

(VIII)

Die Wittig-Horner Olefinierung des Aldehyds VI mit dem Phosphonat der Formel VII und einer Base und gegebenenfalls anschließender Hydrierung sowie anschließender Reduktion der Estergruppe. Oxidation des primären Alkohols, nochmalige Wittig-Horner-Olefinierung mit dem Phosphonat der Formel VII und gegebenenfalls anschließender Hydrierung oder Wittig-Horner Reaktion des Aldehyds VI mit einem Phosphonat der Formel VIII liefert den Ester der allgemeinen Formel IX, wobei

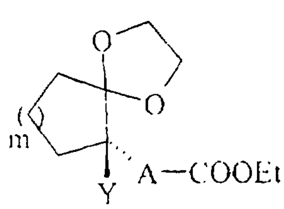

(IX)

m, y und A die oben angegebenen Bedeutungen besitzen. Als Basen kommen beispielsweise Kalium-tert.-butylat, Diazabicyclononan, Diazabicycloundecan oder Natriumhydrid in Frage. Reduktion der Estergruppe, beispielsweise

mit Diisobutylaluminiumhydrid und anschließende Oxidation des erhaltenen primären Alkohols z.B. mit Braunstein oder Collinsreagenz führt zum Aldehyd der Formel X.

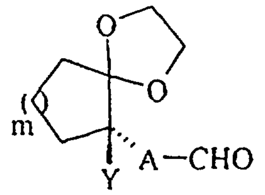

(X)

Die metallorganische Umsetzung des Aldehyds der Formel X mit einem Grignardreagenz der Formel XI, wonn B, D

$$X\text{-}Mg\text{-}B\text{-}D\text{-}R_3 \qquad (XI)$$

und $R_3$ die oben angegebenen Bedeutungen aufweisen und X Chlor, Brom oder Jod bedeutet, führt nach Schutz der Hydroxygruppen (beispielsweise durch Acylierung) und gegebenenfalls Diastereomerentrennung zu den Verbindungen der Formel XII.

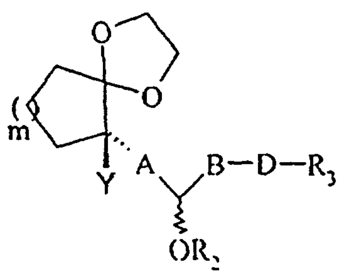

(XII)

Die Herstellung der für die metallorganische Umsetzung benötigten Verbindung der Formel XI erfolgt durch Reaktion des entsprechenden terminalen Hafogenids mit Magnesium. Durch Umsetzung des Ketals XII mit verdünnter Essigsäure und gegebenenfalls Verseifung des Esters und anschließende Silyletherbildung wird das Keton der Formel XIII erhalten:

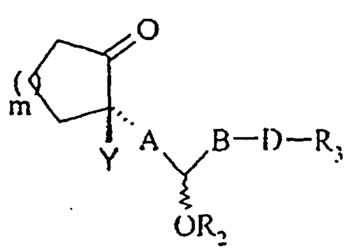

(XIII)

Zu den Verbindungen der Formel XII, worin B eine $CH_2$-Gruppe und D eine -C≡C-Gruppe oder eine CH=CR7-Gruppe bedeutet, gelangt man beispielsweise durch eine metallorganische Umsetzung eines Propargylhalogenids und anschließende Alkylierung mit einem entsprechenden Alkylhalogenid und gegebenenfalls anschließende Lindlar-Hydrierung.

Ein alternativer Aufbau der unteren Kette geht von dem Aldehyd der Formel XIV aus, der aus der Wittig-Horner-Umsetzung des Aldehyds VI und anschließender Reduktion und Oxidation resultierte.

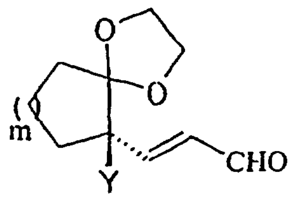

(XIV)

Wittig-Homer-Olefinierung des Aldehyds XIV mit einem Phosphonat der Formel XV

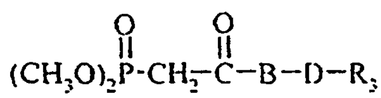

(XV)

und Reduktion des entstandenen Ketons führte dann zum Alkohol der Formel XII der gegebenenfalls in die Diastereomeren getrennt werden kann. Der sich nun anschließende Schutz der Hydroxygruppe, beispielsweise duch Acylierung, Ketalspaltung mit Essigsäure, gegebenenfalls Verseifung des Ester und Silytetherbildung führt zum Keton der Formel XIII.

Die Herstellung der für diese Umsetzung benotigten Phosphonate der allgemeinen Formel XV ist beispielsweise in der DE 42 42 390 beschrieben oder erfolgt in an sich bekannter Weise durch Reaktion eines Alkylhalogenids (herstellbar aus dem entsprechenden Alkohol durch Halogenierung) der allgemeinen Formel XVI

$$\text{Hal-D-R}_3 \qquad \text{(XVI)}$$

mit dem aus Phosphonat der allgemeinen Formel XVII erzeugten Dianion,

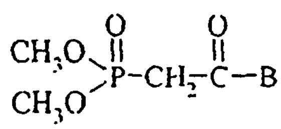

(XVII)

worin B, D und $R_3$ die oben angegebenen Bedeutungen aufweisen.

Ein alternativer Zugang zu den Phosphonaten der allgemeinen Formel XV besteht in der Umsetzung des Anions von Methylphosphonsäuredimethylester mit einem Ester der allgemeinen Formel XVIII,

$$\text{R}_9\text{OOC-B-D-R}_3 \qquad \text{(XVIII)}$$

worin $R_3$, B, D die oben angegebenen Bedeutungen aufweisen und $R_9$ eine Alkylgruppe mit 1-5 C-Atomen bedeutet. Diesen Ester kann man beispielsweise durch Alkylierung mit dem entsprechenden Halogenid erhalten.

Der Einbau der chemisch und metabolisch labilen cis-$\Delta^{6,7}$-Doppelbindung des $LTB_4$ in einen cis-1,2-substituierten Cycloalkylring führt zu einer Stabilisierung, wobei insbesondere durch weitere Derivatisierung der funktionellen Gruppen und/oder strukturelle Veränderungen der unteren Seitenkette, LTB4-Derivate erhalten wurden, die als $LTB_4$-Antagonisten wirken können (DE-A 3917597 und DE-A 42 27 790.6 und DE-A 41 08 351 und DE-A 41 39 886.8 und DE-A 42 42 390).

Es wurde nun gefunden, daß durch Einführung einer Alkylgruppe in die 7-Position und durch Einführung eines Oximethereinheit in der 5,6-Position (Zählweise beginnend beim Caboxyl-C-Atom mit 1 bei Anwendung der $LTB_4$-Nomenklatur) in derartigen Leukotrien $B_4$-Derivaten eine längere Wirkungsdauer größere Selektivität und bessere Wirksamkeit erzielt werden kann.

Die Verbindungen der Formel I wirken antientzündlich, antiallergetisch und antiproliferativ. Die Verbindungen sind außerdem zur Senkung erhöhter Triglyceridspiegel geeignet. Daneben besitzen sie antimykotische Eigenschaften. Folglich stellen die neuen Leukotrien-$B_4$-Derivate der Formel I wertvolle pharmazeutische Wirkstoffe dar. Die Verbindungen der Formel I sind zur topischen und oralen Applikation geeignet

Die neuen Leukotrien-$B_4$-Derivate der Formel I eignen sich in Kombination mit den in der galenischen Pharmazie üblichen Hilfs- und Trägermitteln zur lokalen Behandlung von Erkrankungen der Haut, bei denen Leukotriene eine wichtige Rolle spielen. z. B.: Kontaktdermatitis, Ekzemen der verschiedensten Art, Neurodermatosen, Erythrodermie, Pruritis vulvae et ani, Rosacea, Erythermatodes cutaneus, Psoriasis. Lichen ruber planus et verrucosus und ähnlichen Hauterkrankungen.

Außerdem sind die neuen Leukotrien-$B_4$-Antagonisten zur Behandlung der multiplen Sklerose und der Symptome des Schocks geeignet.

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Salben, Cremes oder Pflaster, überführt.

In den so formulierten Arzneimitteln ist die Wirkstoffkonzentration von der Applikationsform abhängig. Bei Lotionen und Salben wird vorzugsweise eine Wirkstoffkonzentration von 0.0001% bis 3% verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Leukotrien-$B_4$-Derivate auch in Form von Kapseln, Tabletten oder Dragees, die vorzugsweise 0.1 bis 100 mg Wirkstoff enthalten und oral appliziert werden oder in Form von Suspensionen, die vorzugsweise 1-200 mg Wirkstoff pro Dosiseinheit enthaften und rektal appliziert werden auch zur Behandlung von Erkrankungen innerer Organe, bei denen Leukotriene eine wichtige Rolle spielen, wie z. B.: allergische Erkrankungen des Darmtraktes, wie der Colitis ulcerosa und der Colitis granulomatosa.

In diesen neuen Applikationsformen eignen sich die neuen $LTB_4$-Derivate neben der Behandlung von Erkrankungen innerer Organe mit entzündlichen Prozessen auch zur Behandlung von Erkrankungen, bei denen leukotrienabhängig das gesteigerte Wachstum und die Neubildung von Zellen im Vordergrund stehen. Beispiele sind Leukämie (gesteigertes Wachstum weißer Blutkörperchen) oder Artherosklerose (gesteigertes Wachstum glatter Muskelzellen von Blutgefäßen).

Die neuen Leukotrien-$B_4$-Derivate können auch in Kombination, z. B. mit Lipoxygenasehemmem, Cyclooxygenasehemmern, Glukokortikoiden, Prostacyclinagonisten, Thromboxanantagonisten, Leukotrien $D_4$-Antagonisten, Leukotrien $E_4$-Antagonisten, Leukotrien-$F_4$-Antagonisten, Phosphodiesterasehemmern, Calciumantagonisten, PAF-Antagonisten oder anderen bekannten Therapieformen der jeweiligen Erkrankungen, verwendet werden.

Die nachfolgenden Ausführungsbeispiele dienen zur näheren Erläuterung des erfindungsgemäßen Verfahrens.

**Beispiel 1**

<u>(7E)-7-{(2S)-2-[(1E,3E,5S)-5-Hydroxy-9-phenyl-6,6-trimethylen-1,3-nonadien-8-inyl]-2-methylcyclohexyliden}-7-aza-6-oxaheptansäure-methylester</u>

143 mg (2S)-2-[(5S)-5-*tert*-Butyldimethylsilyloxy-9-phenyl-6,6-trimethylen-1,3-nonadien-8-inyl]-2-methylcyclohexanon werden mit 59 mg Hydroxylammoniumsulfat in 3,5 ml Methanol, 3,5 ml Tetrahydrofuran und 3,5 ml Wasser gelöst und 5 Stunden bei Raumtemperatur gerührt. Anschließend wird das Reaktionsgemisch im Vakuum eingeengt und der Rückstand in Ether aufgenommen. Man wäscht mit Wasser und gesättigter Natriumhydrogencarbonat-Lösung, trocknet über Natriumsulfat und engt im Vakuum ein. Der Rückstand wird durch Chromatographie an Kieselgel mit Hexan/Essigester-Gemischen (Gradient: 0-10% Essigester) gereinigt. Ausbeute: 140 mg des Oxims als farbloses Öl.

IR (Film): 3277, 2930, 2857, 1598, 1490, 1462, 1442, 1360, 1255, 1119, 1063, 992, 942, 836, 775, 755, 691 $cm^{-1}$

Zu einer Lösung von 140 mg des vorstehend beschreibenen Oxims in 5 ml N,N-Dimethylformamid gibt man bei Raumtemperatur 17 mg Natriumhydrid (60 proz. Dispersion in Mineralöl) und rührt 1 Stunde bei Raumtemperatur. 82 mg 5-Bromvaleriansäure-methylester werden dazugegeben. Nach zweistündigem Rühren bei Raumtemperatur wird der Ansatz mit Ether verdünnt, mit 10 proz. Citronensäure gewaschen, über Natriumsulfät getrocknet und im Vakuum eingeengt. Der Rückstand wird durch Chromatographie an Kieselgel mit Hexan/Essigester-Gemischen (Gradient: 0-10% Essigester) gereinigt. Ausbeute: 160 mg des Esters als farbloses Öl.

IR (Film): 2940, 2860, 1740, 1660, 1600, 1440, 1360, 1260, 1210, 1170, 990, 840, 780, 760, 690 $cm^{-1}$

Zu einer Lösung von 160 mg des vorstehend beschriebenen Esters in 10 ml Tetrahydrofuran werden 410 mg Tetrabutylammoniumfluorid-Trihydrat gegeben. Nach achtstündigem Rühren bei Raumtemperatur wird der Ansatz mit

Ether verdünnt, mit Wasser gewaschen, über Natriumsulfat getrocknet und im Vakuum eingeengt. Der Rückstand wird durch Chromatographie an Kieselgel mit Hexan/Ether-Gemischen (Gradient: 0-40% Ether) gereinigt. Ausbeute: 82 mg der Titelverbindung als farbloses Öl. Diese Substanz ist die bevorzugte Ausführungsform.
IR (Film): 3480, 2920, 2860, 1740, 1600, 1490, 1440, 1370, 1240, 1170, 1090, 990, 920, 755, 690 $cm^{-1}$

**Beispiel 2**

(7E)-7-{(2S)-2-[(1E,3E,5S)-5-Hydroxy-9-phenyl-6,6-trimethylen-1,3-nonadien-8-inyl]-2-methylcyclohexyliden}-7-aza-6-oxaheptansäure

Zu einer Lösung von 68 mg des in Beispiel 1 beschriebenen Esters in 0,8 ml Methanol und 0,8 ml Tetrahydrofuran gibt man bei Raumtemperatur 0,75 ml 1 N Natronlauge. Nach sechsstündigem Rühren bei Raumtemperatur wird der Ansatz mit 10 proz. Schwefelsäure angesäuert und mit Essigester extrahiert. Die vereinigten organischen Extrakte werden mit gesättigter Natriumchlorid-Lösung gewaschen, über Natriumsulfat getrocknet und im Vakuum eingeengt. Der Rückstand wird durch Chromatographie an Kieselgel mit Hexan/Essigester-Gemischen (Gradient: 0-50% Essigester) gereinigt. Ausbeute: 52 mg der Titelverbindung als farbloses Öl.
IR (Film): 3440, 2920, 2860, 1710, 1600, 1490, 1440, 1370, 1240, 1090, 1040, 990, 920, 840, 760, 695 $cm^{-1}$

**Beispiel 3**

(7E)-7-{(2S)-2-[(1E,3E,5S)-5-Hydroxy-10-phenyl-7,7-trimethylen-1,3-decadien-9-inyl]-2-methylcyclohexyliden}-7-aza-6-oxaheptansäure-methylester

730 mg (2S)-2-[(5S)-5-*tert*-Butyldimethylsilyloxy-10-phenyl-7,7-trimethylen-1,3-decadien-9-inyl]-2-methylcyclohexanon werden mit 290 mg Hydroxylammoniumsulfat in 20 ml Methanol, 20 ml Tetrahydrofuran und 20 ml Wasser 6 Stunden bei Raumtemperatur gerührt. Das Reaktionsgemisch wird im Vakuum eingeengt und der Rückstand in Ether aufgenommen. Man wäscht mit Wasser und gesättigter Natriumhydrogencarbonat-Lösung, trocknet über Natriumsulfat und engt im Vakuum ein. Der Rückstand wird durch Chromatographie an Kieselgel mit Hexan/Essigester-Gemischen (Gradient: 0-10% Essigester) gereinigt. Ausbeute: 700 mg des Oxims als farbloses Öl.
IR (Film): 3280, 2930, 2860, 1740, 1600, 1490, 1470, 1460, 1440, 1370, 1360, 1250, 1105, 1070, 990, 835, 810, 775, 755, 690 $cm^{-1}$
Zu einer Lösung von 200 mg des vorstehend beschriebenen Oxims in 6 ml N,N-Dimethylformamid werden 24 mg Natriumhydrid (60 proz. Dispersion in Mineralöl) gegeben. Nach einstündigem Rühren bei Raumtemperatur wird mit 120 mg 5-Bromvaleriansäure-methylester versetzt. Nach zweistündigem Rühren bei Raumtemperatur wird der Ansatz mit Ether verdünnt, mit 10 proz. Citronensäure gewaschen, über Natriumsulfat getrocknet und im Vakuum eingeengt. Der Rückstand wird durch Chromatographie an Kieselgel mit Hexan/Essigester-Gemischen (Gradient: 0-10% Essigester) gereinigt. Ausbeute: 240 mg des Esters als farbloses Öl.
IR (Film): 2920, 2860, 1740, 1600, 1490, 1440, 1360, 1255, 1200, 1170, 1070, 990, 840, 775, 755, 690 $cm^{-1}$
Zu einer Lösung von 230 mg des vorstehend beschriebenen Esters in 10 ml Tetrahydrofuran werden 580 mg Tetrabutylammoniumfluorid-Trihydrat gegeben. Nach achtstündigem Rühren bei Raumtemperatur wird der Ansatz mit Ether verdünnt, mit Wasser gewaschen, über Natriumsulfat getrocknet und im Vakuum eingeengt. Der Rückstand wird durch Chromatographie an Kieselgel mit Hexan/Ether-Gemischen (Gradient: 0-40% Ether) gereinigt. Ausbeute: 140 mg der Titelverbindung als farbloses Öl.
IR (Film): 3460, 2920, 2860, 1740, 1600, 1490, 1440, 1370, 1250, 1200, 1170, 1100, 1070, 990, 920, 890, 760, 690 $cm^{-1}$

**Beispiel 4**

(7E)-7-{(2S)-2-[(1E,3E,5S)-5-Hydroxy-10-phenyl-7,7-trimethylen-1,3-decadien-9-inyl]-2-methylcyclohexyliden}-7-aza-6-oxaheptansäure

Zu einer Lösung von 100 mg des in Beispiel 3 beschriebenen Esters in 1,1 ml Methanol und 1,1 ml Tetrahydrofuran wird bei Raumtemperatur 1 ml 1 N Natronlauge gegeben. Es wird 6 Stunden bei Raumtemperatur gerührt, mit 10 proz. Schwefelsäure angesäuert und mit Essigester extrahiert. Die vereinigten Extrakte werden mit gesättigter Natriumchlorid-Lösung gewaschen, über Natriumsulfat getrocknet und im Vakuum eingeengt. Der Rückstand wird durch Chromatographie an Kieselgel mit Hexan/Essigester-Gemischen (Gradient: 0-50% Essigester) gereinigt. Ausbeute: 96 mg der Titelverbindung als farbloses Öl.
IR (Film): 3400, 2920, 2860, 1710, 1600, 1490, 1440, 1370, 1240, 1070, 990, 920, 760, 690 $cm^{-1}$

**Beispiel 5**

(7E)-7-{(2S)-2-[(1E,3E,5S)-5-Hydroxy-10-phenyl-6,6-trimethylen-1,3-decadien-9-inyl]-2-methylcyclohexyliden}-7-aza-6-oxaheptansäure-methylester

478 mg (2S)-2-[(5S)-5-*tert*-Butyldimethylsilyloxy-10-phenyl-6,6-trimethylen-1,3-decadien-9-inyl]-2-methylcyclohexanon werden mit 192 mg Hydroxylammoniumsulfat in 13 ml Methanol, 13 ml Tetrahydrofuran und 13 ml Wasser 5 Stunden bei Raumtemperatur gerührt. Anschließend wird das Reaktionsgemisch im Vakuum eingeengt und der Rückstand in Ether aufgenommen. Man wäscht mit Wasser und gesättigter Natriumhydrogencarbonat-Lösung, trocknet über Natriumsulfat und engt im Vakuum ein. Der Rückstand wird durch Chromatographie an Kieselgel mit Hexan/Essigester-Gemischen (Gradient: 0-10% Essigester) gereinigt. Ausbeute: 427 mg des Oxims als farbloses Öl.
IR (Film): 3273, 2929, 2860, 1598, 1490, 1442, 1360, 1252, 1675, 993, 942, 836, 775, 756, 692 $cm^{-1}$

Zu einer Lösung von 416 mg des vorstehend beschriebenen Oxims in 13 ml N,N-Dimethylformamid werden 46 mg Natriumhydrid (60 proz. in Mineralöl) bei Raumtemperatur gegeben. Nach einstündigem Rühren bei Raumtemperatur wird mit 241 mg 5-Bromvaleriansaure-methylester versetzt. Nach zweistündigem Rühren bei Raumtemperatur wird der Ansatz mit Ether verdünnt, mit 10 proz. Citronensäure gewaschen, über Natriumsulfat getrocknet und im Vakuum eingeengt. Der Rückstand wird durch Chromatographie an Kieselgel mit Hexan/Essigester-Gemischen (Gradient: 0-10% Essigester) gereinigt. Ausbeute: 410 mg des Esters als farbloses Öl.
IR (Film): 2929, 2857, 2360, 1741, 1598, 1490, 1442, 1360, 1250, 1168, 1056, 992, 836, 775, 756, 692 $cm^{-1}$

Zu einer Lösung von 386 mg des vorstehend beschrieben Esters in 2,5 ml Tetrahydrofuran werden 393 mg Tetrabutylammoniumfluorid-Trihydrat gegeben. Nach achtstündigem Rühren bei Raumtemperatur wird der Ansatz mit Ether verdünnt, mit Wasser gewaschen, über Natriumsulfat getrocknet und im Vakuum eingeengt. Der Rückstand wird durch Chromatographie an Kieselgel mit Hexan/Ether-Gemischen (Gradient: 0-40% Ether) gereinigt. Ausbeute: 241 mg der Titelverbindung als farbloses Öl.
IR (Film): 3476, 2931, 2840, 1738, 1491, 1442, 1372, 1169, 1070, 993, 757, 692 $cm^{-1}$

**Beispiel 6**

(7E)-7-{(2S)-2-[(1E,3E,5S)-5-Hydroxy-10-phenyl-6,6-trimethylen-1,3-decadien-9-inyl]-2-methylcyclohexyliden}-7-aza-6-oxaheptansäure

Zu einer Lösung von 235 mg des in Beispiel 5 beschriebenen Esters in 10 ml Methanol und 5 ml Tetrahydrofuran werden 9,8 ml 0,5 N Natronlauge bei Raumtemperatur gegeben. Nach sechsstündigem Rühren bei Raumtemperatur wird der Ansatz mit 10 proz. Schwefelsäure angesäuert und mit Essigester extrahiert. Die vereinigten Extrakte werden mit gesättigter Natriumchlorid-Lösung gewaschen, über Natriumsulfat getrocknet und im Vakuum eingeengt. Der Rückstand wird durch Chromatographie an Kieselgel mit Hexan/Essigester-Gemischen (Gradient: 0-50% Essigester) gereinigt. Ausbeute: 227 mg der Titelverbindung als farbloses Öl
IR (Film): 3440, 2930, 2860, 1710, 1600, 1490, 1440, 1370, 1240, 1180, 1090, 1070, 1040, 990, 920, 890, 840, 760, 690 $cm^{-1}$

**Beispiel 7**

(4E)-4-{(2S)-2-[(1E,3E,5S)-5-Hydroxy-10-phenyl-7,7-trimethylen-1,3-decadien-9-inyl]-2-methylcyclohexyliden}-4-aza-3-oxabutansäure

730 mg (2S)-2-[(5S)-5-*tert*-Butyldimethylsilyloxy-10-phenyl-7,7-trimethylen-1,3-decadien-9-inyl]-2-methylcyclohexanon werden mit 290 mg Hydroxylammoniumsulfat in 20 ml Methanol, 20 ml Tetrahydrofuran und 20 ml Wasser 5 Stunden bei Raumtemperatur gerührt. Anschließend wird das Reaktionsgemisch im Vakuum eingeengt und der Rückstand in Ether aufgenommen. Man wäscht mit Wasser und gesättigter Natriumhydrogencarbonat-Lösung, trocknet über Natriumsulfat und engt im Vakuum ein. Der Rückstand wird durch Chromatographie an Kieselgel mit Hexan/Essigester-Gemischen (Gradient: 0-10% Essigester) gereinigt. Ausbeute: 700 mg des Oxims als farbloses Öl.
IR (Film): 3280, 2930, 2860, 1740, 1600, 1490, 1470, 1460, 1440, 1370, 1360, 1250, 1105, 1070, 990, 835, 810, 775, 755, 690 $cm^{-1}$

Zu einer Lösung von 240 mg des vorstehend beschriebenen Oxims in 7 ml N,N-Dimethylformamid werden 28 mg Natriumhydrid (60 proz. Dispersion in Mineralöl) gegeben. Nach einstündigem Rühren bei Raumtemperatur wird mit 120 mg 2-Bromessigsäure-ethylester versetzt. Nach zweistündigem Rühren bei Raumtemperatur wird der Ansatz mit Ether verdünnt, die organische Phase mit 10 proz. Citronensäure gewaschen, über Natriumsulfat getrocknet und im Vakuum eingeengt. Der Rückstand wird durch Chromatographie an Kieselgel mit Hexan/Essigester-Gemischen

(Gradient: 0-10% Essigester) gereinigt. Ausbeute: 230 mg des Esters als farbloses Öl.
IR (Film): 2928, 2856, 2361, 1760, 1738, 1480, 1443, 1373, 1256, 1198, 1103, 933, 890, 836, 775, 756, 692 $cm^{-1}$

Zu einer Lösung von 220 mg des vorstehend beschriebenen Esters in 10 ml Tetrahydrofuran werden 580 mg Tetrabutylammoniumfluorid-Trihydrat gegeben. Nach achtstündigem Rühren bei Raumtemperatur wird der Ansatz mit Ether verdünnt, mit Wasser gewaschen, über Natriumsulfat getrocknet und im Vakuum eingeengt. Der Rückstand wird durch Chromatographie an Kieselgel mit Hexan/Ether-Gemischen (Gradient: 0-40% Ether) gereinigt. Ausbeute: 94 mg der Titelverbindung als farbloses Öl.
Zu einer Lösung von 57 mg des Esters in 1 ml Methanol und 1 ml Tetrahydrofuran werden 1 ml 1 N Natronlauge gegeben. Nach sechsstündigem Rühren bei Raumtemperatur wird der Ansatz mit 10 proz. Schwefelsäure angesäuert und mit Essigester extrahiert. Die vereinigten organischen Extrakte werden mit gesättigter Natriumchlorid-Lösung gewaschen, über Natriumsulfat getrocknet und im Vakuum eingeengt. Der Rückstand wird durch Chromatographie an Kieselgel mit Hexan/Essigester-Gemischen (Gradient: 0-50% Essigester) gereinigt. Ausbeute: 62 mg der Titelverbindung als farbloses Öl.
IR (Film): 3440, 2920, 2860, 1700, 1600, 1490, 1440, 1370, 1310, 1090, 1060, 990, 910, 760, 690 $cm^{-1}$

**Beispiel 8**

<u>(6E)-6-{(2S)-2-[(1E,3E,5S)-5-Hydroxy-10-phenyl-7,7-trimethylen-1,3-decadien-9-inyl]-2-methylcyclohexyliden}-6-aza-5-oxahexansäure-methylester</u>

730 mg (2S)-2-[(5S)-5-*tert*-Butyldimethylsilyloxy-10-phenyl-7,7-trimethylen-13-decadien-9-inyl]-2-methylcyclohexanon werden mit 290 mg Hydroxylammoniumsulfat in 20 ml Methanol, 20 ml Tetrahydrofuran und 20 ml Wasser 6 Stunden bei Raumtemperatur gerührt. Das Reaktionsgemisch wird im Vakuum eingeengt und der Rückstand in Ether aufgenommen. Man wäscht mit Wasser und gesättigter Natriumhydrogencarbonat-Lösung, trocknet über Natriumsulfat und engt im Vakuum ein. Der Rückstand wird durch Chromatographie an Kieselgel mit Hexan/Essigester-Gemischen (Gradient: 0-10% Essigester) gereinigt. Ausbeute: 700 mg des Oxims als farbloses Öl.
IR (Film): 3280, 2930, 2860, 1740, 1600, 1490, 1470, 1460, 1440, 1370, 1360, 1250, 1105, 1070, 990, 835, 810, 775, 755, 690 $cm^{-1}$

Zu einer Lösung von 150 mg des vorstehend beschriebenen Oxims in 5 ml N,N-Dimethylformamid werden 18 mg Natriumhydrid (60 proz. Dispersion in Mineralöl) gegeben. Nach einstündigem Rühren bei Raumtemperatur wird der Ansatz mit 78 mg 4-Brombuttersäure-trimethylorthoester verstetzt und 2 Stunden bei Raumtemperatur gerührt. Man verdünnt mit Ether, wäscht die organische Phase mit 10 proz. Citronensäure, trocknet über Natriumsulfat und engt im Vakuum ein. Der Rückstand wird durch Chromatographie an Kieselgel mit Hexan/Essigester-Gemischen (Gradient: 0-10% Essigester) gereinigt. Ausbeute: 180 mg des Esters als farbloses Öl.
IR (Film): 2964, 2836, 1739, 1440, 1371, 1301, 1259, 1170, 1093, 961, 916 $cm^{-1}$

Zu einer Lösung von 180 mg des vorstehend beschriebenen Esters in 10 ml Tetrahydrofuran werden 470 mg Tetrabutylammoniumfluorid-Trihydrat gegeben. Nach achtstündigem Rühren bei Raumtemperatur wird der Ansatz mit Ether verdünnt, mit Wasser gewaschen, über Natriumsulfat getrocknet und im Vakuum eingeengt. Der Rückstand wird durch Chromatographie an Kieselgel mit Hexan/Ether-Gemischen (Gradient: 0-40% Ether) gereinigt. Ausbeute: 110 mg der Titelverbindung als farbloses Öl.
IR (Film): 3460, 2920, 2860, 1740, 1600, 1490, 1440, 1370, 1320, 1250, 1200, 1170, 1090, 1050, 990, 950, 900, 760, 690 $cm^{-1}$

**Beispiel 9**

<u>(6E)-6-{(2S)-2-[(1E,3E,5S)-5-Hydroxy-10-phenyl-7,7-trimethylen-1,3-decadien-9-inyl]-2-methylcyclohexyliden}-6-aza-5-oxahexansäure</u>

Zu einer Lösung von 75 mg des in Beispiel 8 beschriebenen Esters in 0,8 ml Methanol und 0,8 ml Tetrahydrofuran werden 0,8 ml 1 N Natronlauge gegeben. Nach sechsstündigem Rühren bei Raumtemperatur wird der Ansatz mit 10 proz. Schwefelsäure angesäuert und mit Essigester extrahiert. Die vereinigten organischen Extrakte werden mit gesättigter Natriumchlorid-Lösung gewaschen, über Natriumsulfat getrocknet und im Vakuum eingeengt. Der Rückstand wird durch Chromatographie an Kieselgel mit Hexan/Essigester-Gemischen (Gradient: 0-50% Essigester) gereinigt. Ausbeute: 62 mg der Titelverbindung als farbloses Öl.

IR (Film): 3390, 2920, 1710, 1600, 1490, 1440, 1380, 1370, 1260, 1050, 990, 760, 690 $cm^{-1}$

**Beispiel 10**

(8E)-8-{(2S)-2-[(1E,3E,5S)-5-Hydroxy-10-phenyl-7,7-trimethylen-1,3-decadien-9-inyl]-2-methylcyclohexyliden}-8-aza-7-oxaoctansäure-ethylester

2,14 g (2S)-2-[(5S)-5-*tert*-Butyldimethylsilyloxy-10-phenyl-7,7-trimethylen-1.3-decadien-9-inyll-2-methylcyclohexanon werden mit 860 mg Hydroxylammoniumsulfat in 50 ml Methanol, 50 ml Tetrahydrofuran und 50 ml Wasser 5 Stunden bei Raumtemperatur gerührt. Anschließend wird das Reaktionsgemisch im Vakuum eingeengt und der Rückstand in Ether aufgenommen. Man wäscht mit Wasser und gesättigter Natriumhydrogencarbonat-Lösung, trocknet uber Natriumsulfat und engt im Vakuum ein. Der Rückstand wird durch Chromatographie an Kieselgel mit Hexan/Essigester-Gemischen (Gradient: 0-10% Essigester) gereinigt. Ausbeute: 1.78 g des Oxims als farbloses Öl.
IR (Film): 3280, 2930, 2860, 1740, 1600, 1490, 1470, 1460, 1440, 1370, 1360, 1250, 1105, 1070, 990, 835, 810, 775, 755, 690 $cm^{-1}$

Zu einer Lösung von 200 mg des vorstehend beschnebenen Oxims in 6 ml N,N-Dimethylformamid werden 24 mg Natriumhydrid (60 proz. Dispersion in Mineralöl) gegeben. Nach einstündigem Rühren bei Raumtemperatur wird der Ansatz mit 133 mg 6-Bromhexansäure-ethylester versetzt. Nach zweistündigem Rühren bei Raumtemperatur wird das Reaktionsgemisch mit Ether verdünnt, mit 10 proz. Citronensäure gewaschen, über Natriumsulfat getrocknet und im Vakuum eingeengt. Der Rückstand wird durch Chromatographie an Kieseigel mit Hexan/Essigester-Gemischen (Gradient: 0-10% Essigester) gereinigt. Ausbeute: 260 mg des Esters als farbloses Öl.
IR (Film): 2930, 2878, 1736, 1490, 1463, 1373, 1254, 1187, 1070, 992, 836, 776, 756, 692 $cm^{-1}$

Zu einer Lösung von 260 mg des vorstehend beschriebenen Esters in 12 ml Tetrahydrofuran werden 630 mg Tetrabutylammoniumfluorid-Trihydrat gegeben. Nach achtstündigem Rühren bei Raumtemperatur wird der Ansatz mit Ether verdünnt, mit Wasser gewaschen, über Natriumsulfat getrocknet und im Vakuum eingeengt. Der Rückstand wird durch Chromatographie an Kieselgel mit Hexan/Ether-Gemischen (Gradient: 0-40% Ether) gereinigt. Ausbeute: 160 mg der Titelverbindung als farbloses Öl.
IR (Film): 3460, 2925, 2860, 1740, 1600, 1490, 1440, 1370, 1160, 990, 910, 760, 690 $cm^{-1}$

**Beispiel 11**

(8E)-8-{(2S)-2-[(1E,3E,5S)-5-Hydroxy-10-phenyl-7,7-trimethylen-1,3-decadien-9-inyl]-2-methylcyclohexyliden}-8-aza-7-oxaoctansäure

Zu einer Lösung von 129 mg des in Beispiel 10 beschriebenen Esters in 1,4 ml Methanol und 1,4 ml Tetrahydrofuran werden 1.3 ml 1 N Natronlauge gegeben. Nach sechsstündigem Rühren bei Raumtemperatur wird der Ansatz mit 10 proz. Schwefelsäure angesäuert und mit Essigester extrahiert. Die vereinigten organischen Extrakte werden mit gesättigter Natriumchlorid-Lösung gewaschen, über Natriumsulfat getrocknet und im Vakuum eingeengt. Der Rückstand wird durch Chromatographie an Kieselgei mit Hexan/Essigester-Gemischen (Gradient: 0-50% Essigester) gereinigt. Ausbeute: 110 mg der Titelverbindung als farbloses Öl.
IR (Film): 3440, 2920, 2860, 1710, 1600, 1490, 1440, 1380, 1240, 1160, 1090, 1050, 990, 920, 755, 690 $cm^{-1}$

**Beispiel 12**

(7E)-7-{(2S)-2-[(1E,3E,5S)-5-Hydroxy-10-phenyl-7,7-trimethylen-1,3-decadien-9-inyl]-2-methylcyclohexyliden}-7-aza-3,3-dimethyl-6-oxaheptansäure-methylester

1,3 g (2S)-2-[(5S)-5-*tert*-Butyldimethylsilyloxy-10-phenyl-7,7-trimethylen-1,3-decadien-9-inyl]-2-methylcyclohexanon werden mit 522 mg Hydroxylammoniumsulfat in 30 ml Methanol, 30 ml Tetrahydrofuran und 30 ml Wasser versetzt. Nach fünfstündigem Rühren bei Raumtemperatur wird das Reaktionsgemisch im Vakuum eingeengt und der Rückstand in Ether aufgenommen. Man wäscht mit Wasser und gesättigter Natriumhydrogencarbonat-Lösung, trocknet die organische Phase über Natriumsulfat und engt im Vakuum ein. Der Rückstand wird durch Chromatographie an Kieselgel mit Hexan/Essigester-Gemischen (Gradient: 0-20% Essigester) gereinigt. Ausbeute: 1.14 g des Oxims als farbloses Öl.
IR (Film): 3270, 2930, 2850, 1740, 1660, 1600, 1490, 1470, 1460, 1440, 1390, 1370, 1360, 1250, 1070, 1050, 990, 940, 835, 810, 775, 755, 690 $cm^{-1}$

Zu einer Lösung von 400 mg des vorstehend beschriebenen Oxims in 5 ml N,N-Dimethylformamid werden 47 mg Natriumhydrid (60 proz. Dispersion in Mineralöl) gegeben. Nach einstündigem Rühren bei Raumtemperatur wird der Ansatz mit 265 mg 5-Brom-3,3-dimethylpentansäure-methylester (s. Beispiel 12a) versetzt und 2 Stunden bei Raumtemperatur gerührt. Anschließend wird mit Ether verdünnt, mit 10 proz. Citronensäure gewaschen, über Natriumsulfat getrocknet und im Vakuum eingeengt. Der Rückstand wird durch Chromatographie an Kieselgel mit Hexan/ Essigester-Gemischen (Gradient: 0-10% Essigester) gereinigt Ausbeute: 350 mg des Esters als farbloses Öl.
IR (Film): 2929, 2857, 1738, 1558, 1540, 1506, 1490, 1472, 1361, 1255, 1073, 992, 937, 836, 775, 756, 692 $cm^{-1}$

Zu emer Lösung von 320 mg des vorstehend beschriebenen Esters in 10 ml Tetrahydrofuran werden 779 mg Tetrabutylammoniumfluorid-Trihydrat gegeben. Nach achtstündigem Rühren bei Raumtemperatur wird der Ansatz mit Ether verdünnt, mit Wasser gewaschen, uber Natnumsulfat getrocknet und im Vakuum eingeengt. Der Rückstand wird durch Chromatographie an Kieselgel mit Hexan/Ether-Gemischen (Gradient: 0-40% Ether) gereinigt. Ausbeute: 250 mg der Titelverbindung als farbloses Öl.
IR (Film): 3452, 2929, 2825, 1738, 1658, 1598, 1547, 1513, 1490, 1442, 1371, 1224, 1126, 1042, 991, 925, 757, 692 $cm^{-1}$

**Beispiel 12a)**

<u>5-Brom-3,3-dimethylpentansäure-methylester</u>

18 g Natriumborhydrid werden in 480 ml 2-Propanol suspendiert. Nach 30-stündigem Rühren bei Raumtemperatur wird eine Lösung von 48 g Dimethylglutarsäureanhydrid in 320 ml 2-Propanol dazugetropft. Das Reaktionsgemisch wird 2.5 Stunden unter Rückfluß erhitzt. Anschließend engt man im Vakuum ein, gibt den Rückstand auf Eis, säuert mit konzentrierter Salzsäure auf pH 2 an und rührt 1 Stunde bei Raumtemperatur und 30 Minuten bei 80°C. Man extrahiert mit Ether, wäscht die vereinigten Extrakte mit gesättigter Natriumhydrogencarbonat- und gesättigter Natriumchlorid-Lösung, trocknet über Natriumsulfat und engt im Vakuum ein. Den Rückstand reinigt man durch Vakuumdestillation (150°C/12mbar). Ausbeute: 24 g des Lactons als farbloses Öl. IR (Film): 2960, 2870, 1730, 1600, 1485, 1470, 1405, 1370, 1315, 1255, 1175, 1140, 1080, 1040, 1010, 990, 955, 890, 825, 665 $cm^{-1}$

16 g Bromwasserstoff werden in 49 g Essigsäure bei 10°C-23°C eingeleitet. Anschließend werden 7 g des vorstehend hergestellten Lactons in 5 ml Essigsäure dazugegeben. Nach 72-stündigem Rühren bei Raumtemperatur wird der Ansatz auf Eiswasser gegossen. Die Kristalle werden abgesaugt und in Dichlormethan gelöst. Die Lösung wird über Natriumsulfat getrocknet und im Vakuum eingeengt. Der Rückstand wird durch Kristallisation aus Hexan gereinigt. Ausbeute: 6.9 g des Bromids als weiße Kristalle,
IR (Film): 2970, 2880, 1710, 1460, 1410, 1390, 1370, 1305, 1250, 1180, 1130, 1090, 1040, 990, 950, 665, 630 $cm^{-1}$

Zu einer Lösung von 1 g des vorstehend beschriebenen Bromids in 10 ml Ether wird bei 0°C unter Stickstoff eine etherische Diazomethan-Lösung langsam getropft, bis keine Gasentwicklung mehr erkennbar ist. Anschießend wird der Ansatz im Vakuum eingeengt. Der Rückstand wird durch Chromatographie an Kieselgel mit Hexan/Ether-Gemischen (Gradient: 0-40% Ether) gereinigt. Ausbeute: 1 g der Titelverbindung als farbloses Öl.

**Beispiel 13**

<u>(7E)-7-{(2S)-2-[(1E,3E,5S)-5-Hydroxy-10-phenyl-7,7-trimethylen-1,3-decadien-9-inyl]-2-methylcyclohexyliden}-7-aza-3,3-dimethyl-6-oxa-heptansäure</u>

Zu einer Lösung von 250 mg des in Beispiel 12 beschriebenen Esters in 3 ml Methanol und 4 ml Tetrahydrofuran werden 2,3 ml 1 N Natronlauge gegeben. Nach sechsstündigem Rühren bei Raumtemperatur wird der Ansatz mit 10 proz. Schwefelsäure angesäuert und mit Essigester extrahiert. Die vereinigten Extrakte werden mit gesättigter Natriumchlond-Losung gewaschen, über Natriumsulfat getrocknet und im Vakuum eingeengt. Der Rückstand wird durch Chromatographie an Kieselgel mit Hexan/Essigester-Gemischen (Gradient: 0-50% Essigester) gereinigt. Ausbeute: 218 mg der Titelverbindung als farbloses Öl.
IR (Film): 3400, 2920, 2860, 1710, 1590, 1570, 1490, 1440, 1370, 1240, 1120, 1090, 1040, 990, 920, 890, 755, 690 $cm^{-1}$

**Beispiel 14**

<u>(7E)-7-{(2S)-2-[(1E,3E,5S)-5-Hydroxy-10-phenyl-7,7-trimethylen-1,3-decadien-9-inyl]-2-methylcyclohexyliden}-7-aza-3.6-dioxaheptansäure-*tert*-butylester</u>

1,3 g (2S)-2-[(5S)-5-*tert*-Butyldimethylsilyloxy-10-phenyl-7,7-trimethylen-1,3-decadien-9-inyl]-2-methylcyclohexanon werden mit 522 mg Hydroxylammoniumsulfat in 30 ml Methanol, 30 ml Tetrahydrofuran und 30 ml Wasser 7 Stunden bei Raumtemperatur gerührt. Anschließend wird das Reaktionsgemisch im Vakuum eingeengt und der Rückstand in Ether aufgenommen. Man wäscht mit Wasser und gesättigter Natriumhydrogencarbonat-Lösung, trocknet die organische Phase über Natriumsulfat und engt im Vakuum ein. Der Rückstand wird durch Chromatographie an Kieselgel mit Hexan/Ether-Gemischen (Gradient: 0-20% Ether) gereinigt. Ausbeute: 1.14 g des Oxims als farbloses Öl.

IR (Film): 3270, 2930, 2850, 1740, 1660, 1600, 1490, 1470, 1460, 1440, 1390, 1370, 1360, 1250, 1070, 1050, 990, 940, 835, 810, 775, 755, 690 $cm^{-1}$

Zu einer Lösung von 700 mg des vorstehend beschriebenen Oxims in 10 ml N,N-Dimethylformamid werden 83 mg Natriumhydrid (60 proz. Dispersion in Mineralöl) gegeben. Nach einstündigem Rühren bei Raumtemperatur wird mit 347 mg 2-Bromessigsäure-ethylester versetzt. Nach zweistündigem Rühren bei Raumtemperatur wird der Ansatz mit Ether verdünnt, mit 10 proz. Citronensäure gewaschen, über Natriumsulfat getrocknet und im Vakuum eingeengt. Der Rückstand wird durch Chromatographie an Kieselgel mit Hexan/Ether-Gemischen (Gradient: 0-10% Ether) gereinigt. Ausbeute: 740 mg des Esters als farbloses Öl.
IR (Film): 2928, 2856, 2359, 1760, 1738, 1598, 1490, 1462, 1444, 1376, 1256, 1198, 1103, 993, 891, 836, 775, 756, 692 $cm^{-1}$

Zu einer Lösung von 730 mg des vorstehend beschriebenen Esters in 15 ml Toluol werden bei -60°C unter Stickstoff 2.3 ml Diisobutylaluminiumhydrid (20 proz. in Toluol) gegeben. Nach 40 Minuten werden 1 ml Isopropanol und 1 ml Wasser dazugetropft. Nach zweistündigem, kräftigen Rühren bei Raumtemperatur wird der Niederschlag abgesaugt und gründlich mit Essigester gewaschen. Die vereinigten Filtrate werden im Vakuum eingeengt. Der Rückstand wird durch Chromatographie an Kieselgel mit Hexan/Ether-Gemischen (Gradient: 0-40% Ether) gereinigt. Ausbeute: 600 mg des Alkohols als farbloses Öl.
IR (Film): 3471, 2926, 2860, 1598, 1490, 1443, 1361, 1256, 1044, 992, 938, 836. 775, 756, 692 $cm^{-1}$ Zu einer Lösung von 590 mg des vorstehend beschriebenen Alkohols in 5 ml Toluol werden 0,86 ml 2-Bromessigsäure-*tert*-butylester, 4 ml 25 proz. Natronlauge und 36 mg Tetrabutylammoniumhydrogensulfat gegeben. Nach 16-stündigem Rühren bei Raumtemperatur wird der Ansatz mit Ether verdünnt, mit gesättigter Natriumchlorid-Lösung gewaschen, über Natriumsulfat getrocknet und im Vakuum eingeengt. Der Rückstand wird durch Chromatographie an Kieselgel mit Hexan/Ether-Gemischen (Gradient: 0-20% Ether) gereinigt. Ausbeute: 610 mg des Esters als farbloses Öl.
IR (Film): 2929, 2857, 1750, 1598, 1490, 1461, 1368, 1254, 1225, 1141, 1071, 992, 936, 836, 776, 756, 692 $cm^{-1}$

Zu einer Lösung von 580 mg des vorstehend beschriebenen Esters in 18 ml Tetrahydrofuran werden 1.4 g Tetrabutylammoniumfluorid-Trihydrat gegeben. Nach vierstündigem Rühren bei Raumtemperatur wird der Ansatz mit Ether verdünnt, mit Wasser gewaschen. Natriumsulfat getrocknet und im Vakuum eingeengt. Der Rückstand wird durch Chromatographie an Kieselgel mit Hexan/Ether-Gemischen (Gradient: 0-40% Ether) gereinigt. Ausbeute 410 mg der Titelverbindung als farbloses Öl.
IR (Film): 3477, 2929, 2860, 1748, 1598, 1490, 1443, 1368, 1227, 1141, 1070, 992, 941, 844, 757, 692 $cm^{-1}$

**Beispiel 15**

(7E)-7-{(2S)-2-[(1E,3E,5S)-5-Hydroxy-10-phenyl-7,7-trimethylen-1,3-decadien-9-inyl]-2-methylcyclohexyliden}-7-aza-3,6-dioxaheptansäure

Zu einer Lösung von 390 mg des in Beispiel 14 beschriebenen Esters in 4 ml Methanol und 4 ml Tetrahydrofuran werden 3.6 ml 1 N Natronlauge gegeben. Nach sechsstündigem Rühren bei Raumtemperatur wird der Ansatz mit 10 proz. Schwefelsäure angesäuert und mit Essigester extrahiert. Die vereinigten Extrakte werden mit gesättigter Natriumchlorid-Lösung gewaschen, über Natriumsulfat getrocknet und im Vakuum eingeengt. Der Rückstand wird durch Chromatographie an Kieselgel mit Hexan/Essigester-Gemischen (Gradient: 0-50% Essigester) gereinigt. Ausbeute: 331 mg der Titelverbindung als farbloses Öl.
IR (Film): 3440, 2920, 2860, 1740, 1600, 1490, 1440, 1370, 1240, 1140, 1070, 990, 940, 910, 755, 690 $cm^{-1}$

**Beispiel 16**

4-[(3E)-{(2S)-2-[(1E,3E,5S)-5-Hydroxy-10-phenyl-7,7-trimethylen-1,3-decadien-9-inyl]-2-methylcyclohexyliden}-3-aza-2-oxapropyl]benzoesaure-ethylester

2,1 g (2S)-2-[(5S)-5-*tert*-Butyldimethylsilyloxy-10-phenyl-7,7-trimethylen-1,3-decadien-9-inyl]-2-methylcyclohexanon werden mit 860 mg Hydroxylammoniumsulfat in 50 ml Methanol, 50 ml Tetrahydrofuran und 50 ml Wasser 5 Stunden bei Raumtemperatur gerührt. Anschließend wird das Reaktionsgemisch im Vakuum eingeengt und der Rückstand in Ether aufgenommen. Man wäscht mit Wasser und gesättigter Natriumhydrogencarbonat-Lösung, trocknet die organische Phase über Natriumsulfat und engt im Vakuum ein. Der Rückstand wird durch Chromatographie an Kieselgel mit Hexan/Essigester-Gemischen (Gradient: 0-10% Essigester) gereinigt. Ausbeute: 1,78 g des Oxims als farbloses Ol.
IR (Film): 3280, 2930, 2860, 1740, 1600, 1490, 1470, 1460, 1440, 1370, 1360, 1250, 1105, 1070, 990, 835, 810, 775, 755.690 $cm^{-1}$

Zu einer Lösung von 200 mg des vorstehend beschriebenen Oxims in 6 ml N,N-Dimethylformamid werden

24 mg Natriumhydrid (60 proz. Dispersion in Mineralöl) bei Raumtemperatur gegeben Nach einstündigem Rühren bei Raumtemperatur wird das Reaktionsgemisch mit 145 mg 4-(Brommethyl)benzoesäure-ethylester versetzt und 2 Stunden bei Raumtemperatur gerührt. Anschließend wird der Ansatz mit Ether verdünnt, mit 10 proz. Citronensäure gewaschen, über Natriumsulfat getrocknet und im Vakuum eingeengt. Der Rückstand wird durch Chromatographie an Kieselgel mit Hexan/Essigester-Gemischen (Gradient. 0-10% Essigester) gereinigt. Ausbeute: 200 mg des Esters als farbloses Öl.
IR (Film): 2928, 2856, 1720, 1614, 1490, 1443, 1366, 1275, 1105, 1021, 992, 836, 775, 756, 692 $cm^{-1}$

Zu einer Lösung von 200 mg des vorstehend beschriebenen Esters in 10 ml Tetrahydrofuran werden 470 mg Tetrabutylammoniumfluorid-Trihydrat gegeben. Nach achtstündigem Rühren bei Raumtemperatur wird der Ansatz mit Ether verdünnt, mit Wasser gewaschen über Natriumsulfat getrocknet und im Vakuum eingeengt. Der Rückstand wird durch Chromatographie an Kieselgel mit Hexan/Ether-Gemischen (Gradient: 0-40% Ether) gereinigt. Ausbeute: 128 mg der Titelverbindung als farbloses Öl.
IR (Film): 3480, 2920, 2860, 1720, 1610, 1600, 1420, 1280, 1180, 1110, 1060, 1020, 990, 890, 850, 760, 690 $cm^{-1}$

**Beispiel 17**

4-[(3E)-{(2S)-2-[(1E,3E,5S)-5-Hydroxy-10-phenyl-7,7-trimethylen-1,3-decadien-9-inyl]-2-methylcyclohexyliden}-3-aza-2-oxapropyl]benzoesäure

Zu einer Lösung von 99 mg des in Beispiel 16 beschriebenen Esters in 1 ml Methanol und 1 ml Tetrahydrofuran werden 0,97 ml 1 N Natronlauge gegeben. Nach sechsstündigem Rühren bei Raumtemperatur wird der Ansatz mit 10 proz. Schwefelsäure angesäuert und mit Essigester extrahiert. Die vereinigten organischen Extrakte werden mit gesättigter Natriumchlorid-Lösung gewaschen, über Natriumsulfat getrocknet und im Vakuum eingeengt. Der Rückstand wird durch Chromatographie an Kieselgel mit Hexan/Essigester-Gemischen (Gradient 0-50% Essigester) gereinigt. Ausbeute: 86 mg der Titelverbindung als farbloses Ol
IR (Film): 3440, 2920, 2860, 1740, 1690, 1610, 1600, 1580, 1440, 1420, 1370, 1310, 1240, 1170, 1100, 1050, 1020, 990, 920, 890, 850, 760, 690 $cm^{-1}$

**Beispiel 18**

3-[(3E)-{(2S)-2-[(1E,3E,5S)-5-Hydroxy-10-phenyl-7,7-trimethylen-1,3-decadien-9-inyl]-2-methylcyclohexyliden}-3-aza-2-oxapropyl]benzoesäure-methylester

730 mg (2S)-2-[(5S)-5-*tert*-Butyldimethylsilyloxy-10-phenyl-7,7-trimethylen-1,3-decadien-9-inyl]-2-methylcyclohexanon werden mit 290 mg Hydroxylammoniumsulfat in 20 ml Methanol. 20 ml Tetrahydrofuran und 20 ml Wasser 5 Stunden bei Raumtemperatur gerührt. Anschließend wird das Reaktionsgemisch im Vakuum eingeengt und der Rückstand in Ether aufgenommen. Man wäscht mit Wasser und gesättigter Natriumhydrogencarbonat-Lösung, trocknet über Natriumsulfat und engt im Vakuum ein. Der Rückstand wird durch Chromatographie an Kieselgel mit Hexan/Essigester-Gemischen (Gradient: 0-10% Essigester) gereinigt. Ausbeute: 700 mg des Oxims als farbloses Öl.
IR (Film): 3280, 2930, 2860, 1740, 1600, 1490, 1470, 1460, 1440, 1370, 1360, 1250, 1105, 1070, 990, 835, 810, 775, 755, 690 $cm^{-1}$

Zu einer Lösung von 250 mg des vorstehend beschriebenen Oxims in 5 ml N,N-Dimethylformamid werden 30 mg Natriumhydrid (60 proz. Dispersion in Mineralöl) gegeben. Nach einstündigem Rühren bei Raumtemperatur wird mit 170 mg 3-(Brommethyl)benzoesäure-methylester versetzt. Anschließend wird mit Ether verdünnt, mit 10 proz. Citronensäure gewaschen, über Natriumsulfat getrocknet und im Vakuum eingeengt. Der Rückstand wird durch Chromatographie an Kieselgel mit Hexan/Essigester-Gemischen (Gradient: 0-10% Essigester) gereinigt. Ausbeute: 230 mg des Esters als farbloses Öl.
IR (Film): 2920, 2860, 1730, 1600, 1490, 1440, 1430, 1160, 1290, 1250, 1200, 1100, 1070, 990, 900, 840, 810, 775, 755, 720, 690 $cm^{-1}$

Zu einer Lösung von 230 mg des vorstehend beschriebenen Esters in 10 ml Tetrahydrofuran werden 550 mg Tetrabutylammoniumfluorid-Trihydrat gegeben. Nach achtstündigem Rühren bei Raumtemperatur wird der Ansatz mit Ether verdünnt, mit Wasser gewaschen, über Natriumsulfat getrocknet und im Vakuum eingeengt. Der Rückstand wird durch Chromatographie an Kieselgel mit Hexan/Ether-Gemischen (Gradient: 0-40% Ether) gereinigt. Ausbeute: 121 mg der Titelverbindung als farbloses Öl.
IR (Film): 3480, 2920, 2850, 1720, 1590, 1480, 1440, 1430, 1360, 1280, 1200, 1100, 990, 920, 900, 830, 755, 690 $cm^{-1}$

**Beispiel 19**

3-[(3E)-{(2S)-2-[(1E,3E,5S)-5-Hydroxy-10-phenyl-7,7-trimethylen-1,3-decadien-9-inyl]-2-methylcyclohexyliden}-3-aza-2-oxapropyl]benzoesaure

Zu einer Lösung von 92 mg des in Beispiel 18 beschriebenen Esters in 0,9 ml Methanol und 0,9 ml Tetrahydrofuran werden 0.9 ml 1 N Natronlauge gegeben. Nach sechsstündigem Rühren bei Raumtemperatur wird mit 10 proz. Schwefelsäure angesäuert und mit Essigester extrahiert. Die vereinigten organischen Extrakte werden mit gesättigter Natriumchlorid-Lösung gewaschen, über Natriumsulfat getrocknet und im Vakuum eingeengt. Der Rückstand wird durch Chromatographie an Kieselgel mit Hexan/Essigester-Gemischen (Gradient. 0-50% Essigester) gereinigt. Ausbeute: 93 mg der Titelverbindung als farbloses Ol.
IR (Film): 3400, 2920, 2860. 1690, 1610, 1590, 1490, 1440, 1370, 1260, 1200, 1100, 1040, 990, 830, 755, 690, 660, 650 $cm^{-1}$

**Beispiel 20**

4-[(3E)-{(2S)-2-[(1E,3E,5S)-5-Hydroxy-10-phenyl-7,7-trimethylen-1,3-decadien-9-inyl]-2-methylcyclohexyliden}-3-aza-2-oxapropyl]-1,3-thiazol

2,1 mg (2S)-2-[(5S)-5-*tert*-Butyldimethylsilyloxy-10-phenyl-7,7-trimethylen-1,3-decadien-9-inyl]-2-methylcyclohexanon werden mit 860 mg Hydroxylammoniumsulfat in 50 ml Methanol, 50 ml Tetrahydrofuran und 50 ml Wasser 5 Stunden bei Raumtemperatur gerührt. Anschließend wird das Reaktionsgemisch im Vakuum eingeengt und der Rückstand in Ether aufgenommen. Man wäscht mit Wasser und gesättigter Natriumhydrogencarbonat-Lösung, trocknet über Natriumsulfat und engt im Vakuum ein. Der Rückstand wird durch Chromatographie an Kieselgel mit Hexan/Essigester-Gemischen (Gradient: 0-10% Essigester) gereinigt. Ausbeute: 1,78 mg des Oxims als farbloses Öl.
IR (Film): 3280, 2930, 2860, 1740, 1600, 1490, 1470, 1460, 1440, 1370, 1360, 1250, 1105, 1070, 990, 835, 810, 775, 755, 690 $cm^{-1}$

Zu einer Lösung von 200 mg des vorstehend beschrieben Oxims in 5 ml N,N-Dimethylformamid werden 48 mg Natriumhydrid (60 proz. Dispersion in Mineralöl) gegeben. Nach einstündigem Rühren bei Raumtemperatur wird mit 240 mg 4-(Chlormethyl)thiazol-2-carbonsaure-ethylester versetzt und 2 Stunden bei Raumtemperatur geruhrt. Anschließend wird mit Ether verdunnt. die organische Phase mit 10 proz. Citronensäure gewaschen, über Natriumsulfat getrocknet und im Vakuum eingeengt. Der Rückstand wird durch Chromatographie an Kieselgei mit Hexan/Essigester-Gemischen (Gradient: 0-10% Essigester) gereinigt. Ausbeute: 247 mg des Esters als farbloses Ol.
IR (Film): 2928, 2856, 1712, 1598, 1490, 1462, 1443, 1360, 1253, 1071, 992, 876, 836, 775, 756, 692 $cm^{-1}$

Zu einer Lösung von 247 mg des vorstehend beschriebenen Esters in 12 ml Tetrahydrofuran werden 630 mg Tetrabutylammoniumfluorid-Trihydrat gegeben. Nach achtstündigem Rühren bei Raumtemperatur wird der Ansatz mit Ether verdünnt, mit Wasser gewaschen. über Natnumsulfat getrocknet und im Vakuum eingeengt. Der Rückstand wird durch Chromatographie an Kieselgel mit Hexan/Ether-Gemischen (Gradient: 0-40% Ether) gereinigt. Ausbeute: 82 mg der Titelverbindung als farbloses Öl.
IR (Film): 3400, 2920, 2860, 1740, 1720, 1600, 1490, 1440, 1420, 1390, 1300, 1100, 1070, 1060, 990, 920, 880, 840, 760, 690 $cm^{-1}$

**Beispiel 20a)**

4-(Chlormethyl)thiazol-2-carbonsäure-ethylester

500 mg Oxalsäure-ethylester-thioamid und 476 mg 1,3-Dichloraceton werden in 10 N.N-Dimethylformamid gelöst und 27 Stunden unter Rückfluß erhitzt. Anschließend wird mit Ether verdünnt, mit Wasser gewaschen, über Natriumsulfat getrocknet und im Vakuum eingeengt. Als Rückstand verbleiben 610 mg der Titelverbindung als farbloses Öl
IR (Film): 3105, 2983, 2359, 1716, 1507, 1459, 1391, 1368, 1303, 1255, 1140, 1090, 1017, 970, 862, 758, 715, 656 $cm^{-1}$

**Beispiel 21**

(6E)-6-{(2S)-2-[(1E,3E,5S)-5-Hydroxy-10-phenyl-7,7-trimethylen-13-decadien-9-inyl]-2-methylcyclohexyliden}-6-aza-5-oxa-1,1,1-trifluorohexan

2.1 g (2S)-2-[(5S)-5-*tert*-Butyldimethylsilyloxy-10-phenyl-7,7-trimethylen-1,3-decadien-9-inyl]-2-methylcyclohexanon werden mit 860 mg Hydroxylammoniumsulfat in 50 ml Methanol, 50 ml Tetrahydrofuran und 50 ml Wasser 5 Stunden bei Raumtemperatur gerührt. Anschließend wird das Reaktionsgemisch im Vakuum eingeengt und der Rückstand in Ether aufgenommen. Man wascht mit Wasser und gesättigter Natriumhydrogencarbonat-Lösung, trocknet über Natriumsulfat und engt im Vakuum ein. Der Rückstand wird durch Chromatographie an Kieselgel mit Hexan/Essigester-Gemischen (Gradient: 0-10% Essigester) gereinigt. Ausbeute: 1,78 mg des Oxims als farbloses Öl.
IR (Film): 3280, 2930, 2860, 1740, 1600, 1490, 1470, 1460, 1440, 1370, 1360, 1250, 1105, 1070, 990, 835, 810, 775, 755, 690 $cm^{-1}$

Zu einer Lösung von 150 mg des vorstehend beschriebenen Oxims in 5 ml N,N-Dimethylformamid werden 18 mg Natriumhydrid (60 proz. Dispersion in Mineralöl) gegeben. Nach einstündigem Rühren bei Raumtemperatur wird das Reaktionsgemisch mit 110 mg 4,4,4-Trifluor-1-iodbutan versetzt und 2 Stunden bei Raumtemperatur gerührt. Anschließend wird mit Ether verdünnt, mit 10 proz. Citronensäure gewaschen, über Natriumsulfat getrocknet und im Vakuum eingeengt. Der Rückstand wird durch Chromatographie an Kieselgel mit Hexan/Essigester-Gemischen (Gradient: 0-10% Essigester) gereinigt. Ausbeute: 170 mg des Esters als farbloses Öl.
IR (Film): 2929, 2887, 1490, 1443, 1373, 1334, 1253, 1155, 1071, 1025, 991, 836, 775, 756, 691 $cm^{-1}$

Zu einer Lösung von 170 mg des vorstehend beschriebenen Esters in 10 ml Tetrahydrofuran werden 430 mg Tetrabutylammoniumfluorid-Trihydrat gegeben. Nach achtstündigem Rühren bei Raumtemperatur wird der Ansatz mit Ether verdünnt, mit Wasser gewaschen uber Natriumsulfat getrocknet und im Vakuum eingeengt. Der Rückstand wird durch Chromatographie an Kieselgel mit Hexan/Ether-Gemischen (Gradient: 0-40% Ether) gereinigt. Ausbeute: 130 mg der Titelverbindung als farbloses Öl.
IR (Film): 3440, 2970, 2860, 1740, 1660, 1600, 1490, 1440, 1370, 1330, 1310, 1250, 1230, 1150, 1070, 1020, 990, 910, 890, 830, 755, 690, 660 $cm^{-1}$

**Beispiel 22**

(5E)-5-{(2S)-2-[(1E,3E,5S)-5-Hydroxy-9-phenyl-6,6-trimethylen-1,3-nonadien-8-inyl]-2-methylcyclohexyliden}-5-aza-1,1-dimethoxy-4-oxapentan

1,4 g (2S)-2-[(5S)-5-*tert*-Butyldimethylsilyloxy-9-phenyl-6,6-trimethylen-1,3-nonadien-8-inyl]-2-methylcyclohexanon werden mit 591 mg Hydroxylammoniumsulfat in 35 ml Methanol, 35 ml Tetrahydrofuran und 35 ml Wasser 5 Stunden bei Raumtemperatur gerührt. Anschließend wird das Reaktionsgemisch im Vakuum eingeengt und der Rückstand in Ether aufgenommen Man wäscht mit Wasser und gesättigter Natriumhydrogencarbonat-Lösung, trocknet die organische Phase über Natriumsulfat und engt im Vakuum ein. Der Rückstand wird durch Chromatographie an Kieselgel mit Hexan/Essigester-Gemischen (Gradient: 0-10% Essigester) gereinigt. Ausbeute: 1,36 mg des Oxims als farbloses Öl
IR (Film): 3277, 2930, 2857, 1598, 1490, 1462, 1360, 1255, 1120, 1063, 992, 942, 836, 775, 755, 691 $cm^{-1}$

Zu einer Lösung von 300 mg des vorstehend beschriebenen Oxims in 6 ml N,N-Dimethylformamid werden 36 mg Natriumhydrid (60 proz. Dispersion in Mineralöl) gegeben. Nach einstündigem Rühren bei Raumtemperatur wird der Ansatz mit 223 mg 3-Brompropionaldehyd-dimethylacetal versetzt und 2 Stunden bei Raumtemperatur gerührt. Anschließend wird das Reaktionsgemisch mit Ether verdünnt, mit 10 proz. Citronensäure gewaschen, über Natriumsulfat getrocknet und im Vakuum eingeengt.

Der Rückstand wird durch Chromatographie an Kieselgel mit Hexan/Essigester-Gemischen (Gradient: 0-10% Essigester) gereinigt. Ausbeute: 304 mg des Esters als farbloses Öl.
IR (Film): 2931, 2857, 1653, 1558, 1506, 1490, 1443, 1388, 1254, 1191, 1125, 1057, 992, 836, 775, 756, 692 $cm^{-1}$

Zu einer Lösung von 300 mg des vorstehend beschriebenen Esters in 10 ml Tetrahydrofuran werden 797 mg Tetrabutylammoniumfluorid-Trihydrat gegeben. Nach achtstündigem Rühren bei Raumtemperatur wird der Ansatz mit Ether verdünnt, mit Wasser gewaschen, über Natriumsulfat getrocknet und im Vakuum eingeengt. Der Rückstand wird durch Chromatographie an Kieselgel mit Hexan/Ether-Gemischen (Gradient: 0-40% Ether) gereinigt Ausbeute: 209 mg der Titelverbindung als farbloses Öl.
IR (Film): 3460, 2930, 2860, 1650, 1620, 1600, 1490, 1440, 1390, 1370, 1190, 1130, 1070, 1060, 990, 920, 760, 690 $cm^{-1}$

**Beispiel 23**

(7E)-7-{(2S)-2-[(1E,3E,5S)-5-Hydroxy-10-phenyl-7,7-trimethylen-1,3-decadien-9-inyl]-2-methylcyclohexyliden}-7-aza-6-oxaheptansäure-5-tetrazolylamid

2,1 g (2S)-2-[(5S)-5-*tert*-Butyldimethylsilyloxy-10-phenyl-7,7-trimethylen-1,3-decadien-9-inyl]-2-methylcyclohexanon werden mit 860 mg Hydroxylammoniumsulfat in 50 ml Methanol, 50 ml Tetrahydrofuran und 50 ml Wasser

13 Stunden bei Raumtemperatur gerührt. Anschließend wird das Reaktionsgemisch im Vakuum eingeengt und der Rückstand in Ether aufgenommen. Man wäscht mit Wasser und gesättigter Natriumhydrogencarbonat-Lösung, trocknet über Natriumsulfat und engt im Vakuum ein. Der Rückstand wird durch Chromatographie an Kieselgel mit Hexan/Essigester-Gemischen (Gradient 0-10% Essigester) gereinigt. Ausbeute: 1.8 g des Oxims als farbloses Öl.
IR (Film): 3270, 2930, 2850, 1740, 1660, 1600, 1490, 1470, 1460, 1440, 1390, 1370, 1360, 1250, 1070, 1050, 990, 940, 835, 810, 775, 755, 690 $cm^{-1}$

Zu einer Lösung von 330 mg des vorstehend beschriebenen Oxims in 7 ml N,N-Dimethylformamid werden 40 mg Natriumhydrid (60 proz. Dispersion in Mineralöl) gegeben. Nach einstündigem Rühren bei Raumtemperatur wird der Ansatz mit 191 mg 5-Bromvaleriansäure-methylester versetzt und 2 Stunden bei Raumtemperatur gerührt. Anschließend wird das Reaktionsgemisch mit Ether verdünnt, mit 10 proz.

Citronensäure gewaschen, über Natriumsulfat getrocknet und im Vakuum eingeengt. Der Rückstand wird durch Chromatographie an Kieselgel mit Hexan/Essigester-Gemischen (Gradient: 0-10% Essigester) gereinigt. Ausbeute: 380 mg des Esters als farbloses Öl.
IR (Film): 2927, 2360, 1738, 1598, 1490, 1435, 1361, 1250, 1168, 1070, 992, 836, 776, 756, 692 $cm^{-1}$
Zu einer Lösung von 380 mg des vorstehend beschriebenen Esters in 3 ml Methanol und 3 ml Tetrahydrofuran werden 2,5 ml 1 N Natronlauge gegeben. Nach 16-stündigem Rühren bei Raumtemperatur wird der Ansatz mit 1 N Schwefelsäure auf pH 5 angesäuert und mit Ether extrahiert Die vereinigten organischen Extrakte werden mit gesättigter Natriumchlorid-Lösung gewaschen, über Natriumsulfat getrocknet und im Vakuum eingeengt. Der Rückstand wird durch Chromatographie an Kieselgel mit Hexan/Essigester-Gemischen (Gradient: 0-50% Essigester) gereinigt. Ausbeute: 280 mg der Säure als farbloses Öl
IR (Film): 2926, 1713, 1598, 1490, 1443, 1372, 1254, 1070, 992, 938, 836, 775, 756, 691 $cm^{-1}$

Zu einer Lösung von 260 mg der vorstehend beschriebenen Säure in 1,5 ml Tetrahydrofuran werden unter Stickstoff 50 mg 5-Aminotetrazol und anschließend 100 mg N,N'-Dicyclihexylcarbodiimid in 0,6 ml Tetrahydrofuran gegeben. Nach 20 Stunden bei Raumtemperatur wird der Niederschlag abgesaugt und mit Essigester gewaschen. Die vereinigten Filtrate werden im Vakuum eingeengt. Der Rückstand wird durch Chromatographie an Kieselgel mit Hexan/Essigester/Methanol-Gemischen (Gradient: 0-100% Essigester. 0-10% Methanol) gereinigt. Ausbeute: 260 mg der Amids als farbloses Öl.
IR (Film): 2930, 2860, 1700, 1630, 1600, 1540, 1440, 1400, 1370, 1310, 1250, 1060, 990, 920, 840, 810, 780, 755, 740, 690, $cm^{-1}$

Zu einer Lösung von 260 mg des vorstehend beschriebenen Amids in 14 ml Tetrahydrofuran werden 975 mg Tetrabutylammoniumfluorid-Trihydrat gegeben. Nach 8 Stunden bei Raumtemperatur wird der Ansatz mit Ether verdünnt, mit Wasser gewaschen, über Natriumsulfat getrocknet und im Vakuum eingeengt. Der Rückstand wird durch Chromatographie an Kieselgel mit Hexan/Ether-Gemischen (Gradient: 0-100% Ether) gereinigt. Ausbeute: 131 mg der Titelverbindung als farbloses Öl.
IR (Film): 3220, 2930, 2860, 1700, 1630, 1590, 1540, 1490, 1450, 1400, 1370, 1310, 1250, 1200, 1130, 1090, 1040, 990, 890, 840, 760, 740, 690 $cm^{-1}$

**Beispiel 24**

<u>(7E)-7-{(2S)-2-[(1E,3E,5S)-6,6-Dimethyl-5-hydroxy-9-phenoxy-1,3-nonadienyl]-2-methylcyclohexyliden}-7-aza-3,3-dimethyl-6-oxaheptansäure-methylester</u>

485 mg (2S)-2-[(5S)-5-*tert*-Butyldimethylsilyloxy-6,6-dimethyl-9-phenoxy-1,3-nonadienyl]-2-methylcyclohexanon werden mit 295 mg Hydroxylammoniumsulfat in 10 ml Methanol. 10 ml Tetrahydrofuran und 10 ml Wasser 5 Stunden bei Raumtemperatur gerührt. Anschließend wird das Reaktionsgemisch im Vakuum eingeengt und der Rückstand in Ether aufgenommen Man wäscht mit Wasser und gesättigter Natriumhydrogencarbonat-Lösung, trocknet über Natriumsulfat und engt im Vakuum ein. Der Rückstand wird durch Chromatographie an Kieselgel mit Hexan/Essigester-Gemischen (Gradient: 0-10% Essigester) gereinigt. Ausbeute: 347 mg des Oxims als farbloses Öl.
IR (Film): 3278, 2930, 2840, 1652, 1601, 1587, 1497, 1471, 1386, 1361, 1301, 1247, 1171, 1109, 1062, 993, 942, 836, 775, 753, 691, 666 $cm^{-1}$

Zu einer Lösung von 331 mg des vorstehend beschriebenen Oxims in 5 ml N,N-Dimethylformamid werden 40 mg Natriumhydrid (60 proz. Dispersion in Mineralöl) gegeben. Nach einstündigem Rühren bei Raumtemperatur wird der Ansatz mit 223 mg 5-Brom-3,3-dimethylpentansäure-methylester (Beispiel 12a) versetzt und 2 Stunden bei Raumtemperatur gerührt Anschließend wird das Reaktionsgemisch mit Ether verdünnt, mit 10 proz. Citronensaure gewaschen uber Natriumsulfat getrocknet und im Vakuum eingeengt. Der Rückstand wird durch Chromatographie an Kieselgel mit Hexan/Essigester-Gemischen (Gradient: 0-10% Essigester) gereinigt. Ausbeute: 348 mg des Esters als farbloses Ol.
IR (Film): 2956, 2840, 1738, 1600, 1498, 1471, 1386, 1247, 1110, 1043, 992, 836, 775, 753, 691, 666 $cm^{-1}$

Zu einer Lösung von 339 mg des vorstehend beschriebenen Esters in 10 ml Tetrahydrofuran werden 4,1 g Tetrabutylammoniumfluorid-Trihydrat gegeben. Nach achtstündigem Rühren bei Raumtemperatur wird der Ansatz mit Ether verdünnt, mit Wasser gewaschen, über Natriumsulfat getrocknet und im Vakuum eingeengt. Der Rückstand wird durch Chromatographie an Kieselgel mit Hexan/Ether-Gemischen (Gradient: 0-40% Ether) gereinigt Ausbeute 220 mg der Titelverbindung als farbloses Öl.
IR (Film): 3500, 2932, 2850, 2358, 1738, 1600, 1586, 1498, 1470, 1387, 1246, 1172, 1040, 991, 928, 754, 692 $cm^{-1}$

**Beispiel 25**

(7E)-7-{(2S)-2-[(1E,3E,5S)-6,6-Dimethyl-5-hydroxy-9-phenoxy-1,3-nonadienyl]-2-methylcyclohexyliden}-7-aza-3,3-dimethyl-6-oxaheptansäure

Zu einer Lösung von 218 mg des in Beispiel 24 beschriebenen Esters in 2 ml Methanol und 2 ml Tetrahydrofuran werden 1,9 ml 1 N Natronlauge gegeben. Nach sechsstündigem Rühren bei Raumtemperatur wird der Ansatz mit 10 proz. Schwefelsäure angesäuert und mit Essigester extrahiert. Die vereinigten organischen Extrakte werden mit gesättigter Natriumchlorid-Lösung gewaschen, über Natriumsulfat getrocknet und im Vakuum eingeengt. Der Rückstand wird durch Chromatographie an Kieselgel mit Hexan/Essigester-Gemischen (Gradient: 0-50% Essigester) gereinigt. Ausbeute: 156 mg der Titelverbindung ais farbloses Öl
IR (Film): 3440, 2960, 2880, 1710, 1600, 1580, 1500, 1470, 1375, 1260, 1190, 1160, 1040, 990, 930, 920, 760, 690 $cm^{-1}$

**In vivo Testsystem**

(i). Herstellung von humanen polymorphnukleären Leukozyten (PMN)

PMNs von gesunden Freiwilligen werden aus heparinisiertem venösem Blut durch Dextran - Sedimentation und anschließender Zentrifugation über Ficoll-Histopaque® isoliert. Die verbleibenden Erytrozyten werden durch hypotonische Lyse in 0,2 - prozentiger Natriumchlorid - Lösung beseitigt. Die PMNs werden in Hank's balanced salt solution (HBSS) resuspendiert und mit Ei - Albumin von Hühnern (OVA) oder Rinder - Serum - Albumin (BSA) versetzt.

(ii). LTB4 - Rezeptor - Kompetitions - Bindungsversuch

Humane PMNs werden mit Tritium markiertem Leukotrien $B_4$ ($LTB_4$) in Gegenwart oder Abwesenheit von den getesteten Substanzen in Konzentrationen von 10 µmol/l bis 0,05 mmol/l in HBSS zusammen mit OVA inkubiert. Zell - gebundenes, tritiummarkiertes $LTB_4$ wird von den freien Liganden durch Vakuum - Filtration durch einen Glasfaser - Filter getrennt und in einem Szintillations - Meßgerät gemessen. Die nicht spezifische Bindung von tritiummarkiertem $LTB_4$ wird in Gegenwart von einem Überschuß an unmarkiertem $LTB_4$ (500 nmol/l) bestimmt. Der Kompetitionsfaktor (CF) wird aus dem Verhältnis von der Konzentration der Substanz zu der Konzentration des LTB4 errechnet, was zu einer 50 - prozentigen Reduktion der tritiummarkierten $LTB_4$ Rezeptor Bindung führt.

(iii). $LTB_4$ - induzierter Chemotaxis - Versuch

Der Chemotaxis - Versuch wird mit modifizierten Boyden - Kammern ausgeführt, die aus Transwell® Modulen mit polyvinylpyrrolidon - ummantelten Polykarbon - Filtern mit einer Porengrößen von 3 µm besteht. Der obere Kammerteil enthält die humanen PMNs in HBSS, welches mit BSA oder OVA ergänzt ist. Der untere Kammerteil wird allein mit Puffer oder mit dem chemotaktisch aktiven Leukotrien $B_4$ ($LTB_4$) in einer Konzentration im Rahmen von 1 nmol/l bis 100 nmol/l in Gegenwart oder Abwesenheit von der Testsubstanz hinzugegeben. Die Kammer wird für 60 Minuten in wassergesättigter Atmosphäre mit 5 % Kohlendioxid inkubiert. Die Anzahl der PMNs, die in den unteren Kammerteil gewandert sind, wird durch die Messung der Aktivität des Enzyms. Myeloperoxidase (MPO) in einem kalibrierten Versuch bestimmt. Die Enzymaktivität wird spektrometrisch (450 nm) durch Bestimmung der Rate an $H_2O_2$ - abhängiger Oxidation vom aromatischen Amin 3,3,5,5'-Tetramethylbenzidin (TMB) gemessen.
Der $EC_{50}$ - Wert wird graphisch durch die nicht lineare Regressionkurve bestimmt. Der KB - Wert beschreibt das Vermögen des kompetitiven Antagonisten. Der $K_B$ - Wert wird als die Antagonisten - Konzentration ermittelt, die erforderlich ist, den $EC_{50}$ - Wert des Agonisten um den Faktor 2 anzuheben.
Der $K_B$ - Wert wird wie folgt berechnet:

$$K_B = [LTB_4 \text{ - Rezeptor - Antagonist}] / (DR-1)$$

(DR = ist das Verhältnis von der $LTB_4$ Konzentration, die für die halb - maximale Stimulation in Gegenwart des Antagonisten benötigt wird. zu der $LTB_4$ Konzentration, die für die halb - maximale Stimulation in Abwesenheit des Antagonisten benötigt wird.)

(iv). $LTB_4$/Iloprost - induzierte Hautentzündung in den Ohren von Mäusen

Weibliche NMRI - Mäuse mit einem Gewicht von 26 bis 28 g und einem Alter von 5 bis 6 Wochen werden für dieses *in vivo* Experiment verwendet. Zehn Tiere pro Gruppe werden nach dem Zufallsprinzip in die verschiedenen Behandlungsgruppen eingeteilt und einzeln gehalten. Die Tiere hatten freien Zugang zum Futter und Wasser. Um die orale Aufnahme von topisch zu applizierendem $LTB_4$/Iloprost Lösungen zu vermeiden, werden Sperr - Halsbänder um den Hals der Tiere unter Ether - Narkose kurz vor der topischen Applikation befestigt.
Leukotrien B4 ($LTB_4$) und das stabilen Prostacyclin - Derivat Iloprost wird in Ethanol/Isopropylmyristat (95 + 5 v/v) bei einer Konzentration von 0,003 % (w/v) gelöst 10 µl der $LTB_4$/Iloprost - Lösung wird topisch auf der äußeren Oberfläche eines jeden Ohres (Fläche etwa 1 $cm^2$/Ohr) verabreicht. Dieses entspricht einer Dosis von 0,3µg pro Ohr oder etwa 0,3 µg pro $cm^2$. Tiere, die allein mit $LTB_4$/Iloprost- Lösung behandelt werden, entwickeln die typischen Merkmale einer entzündeten Haut unter Bildung von Ödemen und Infiltration von Neutrophilen. Diese Tiere dienen als Positiv - Kontrolle. Tiere, die allein mit Ethanol/Isopropylmyristat (95 + 5 v/v) auf der äußeren Oberfläche eines jeden Ohres (Fläche etwa 1 $cm^2$/Ohr) behandelt werden, dienen als Negativ - Kontrolle.

Der Effekt des LTB4 - Rezeptor - Antagonisten auf die $LTB_4$/Iloprost induzierte Entzündungsreaktion wird entweder bei einer topischen oder bei einer intragastralen Verabreichung von der Testsubstanz bestimmt.

Für die topische Applikation wird die Testsubstanz in einer LTB4/Iloprost - Lösung in verschiedenen Konzentration gelöst. 10 µl dieser Lösung wird topisch auf die äußere Oberfläche des Ohr aufgetragen.
Für die intragastrale Applikation wird der LTB4 - Rezeptor - Antagonist in Ethanol gelöst. Direkt nach der topische Verabreichung mit LTB4/Iloprost wird der LTB4 - Rezeptor - Antagonist oder nur das Lösungsmittel intragastral bei verschiedenen Dosen mit Hilfe einer Sonde verabreicht Die höchste Endkonzentration von Ethanol ist 3 %. Die Menge an Ethanol nimmt mit weiteren Verdünnungsschritten ab.

Die Tiere werden 24 Stunden nach dem Setzen der entzündlichen Reaktion getötet. Die Ohren werden abgetrennt, gewogen, schock - gefroren und für weitere Untersuchungen gelagert. Die Peroxidase Aktivität wird spektrometrisch in dem Homogenat von der Ohrhaut bestimmt. Das Gewebe wird in HTAB-Puffer (0,5% Hexadecyltrimethylammoniumbromid (w/v) in $10^{-3}$ mol/l 3-[N-morpholino]propansulfonsäure mit pH 7,0) für 20 Sekunden mit einem Polytron® PT 3000 (Kinematica AG, Schweiz) bei einer Rotation von 30000 Umdrehungen pro Minute homogenisiert. Das Homogenat wird für 20 Minuten bei 10 °C und bei 14500 Umdrehungen pro Minute (20000g) in einer Sorvall RC2-B Zentrifuge (SM-24 Rotor) zentrifugiert. Der wäßrige Überstand wird abgesaugt und auf seine Peroxidase Aktivität bei einer Verdünnung von 1 zu 50 in HTAB-Puffer ausgetestet. Die Peroxidase Aktivität wird durch photomethrische Messung der Rate an $H_2O_2$ - abhängigen Oxidation des aromatischen Amins 3,3',5,5'-Tetramethylbenzidin (TMB) bestimmt. In einer 96 - Loch - Mikrotiterplatte werden die verdünnten Überstände mit TMB - Lösung und Hydrogenperoxid inkubiert (Lösung von 6,5 mg 3,3',5,5'-Tetramethylbenzidindihydrochlorid in 1 ml Dimethylsulfoxid (DMSO); 1: 100 (v/v) gelöst mit 0,1 mol/l Natrium - Acetat - Zitrat - Puffer, pH 6,0, endgültige Konzentration in der Inkubationsmixtur: $1,57 \cdot 10^{-4}$ mol/l) (Hydrogenperoxid 30% $H_2O_2$ 1 : 16860 (v/v) gelöst mit 0.1 mol/l Natrium - Acetat - Zitrat - Puffer, pH 6,0, endgültige Konzentration in der Inkubationsmixtur: $4.93 \cdot 10^{-5}$ mol/l). Nach 30 Minuten bei Raumtemperatur wird die Reaktion durch Zugabe von 0,5 mol/l Schwefelsäure gestoppt. Die Extinktion wird bei 450 nm (maximale Absorption) in einem Mikrotiter - Platten Meßgerät bestimmt.

## Patentansprüche

1. Leukotrien-$B_4$-Derivate der allgemeinen Formel I,

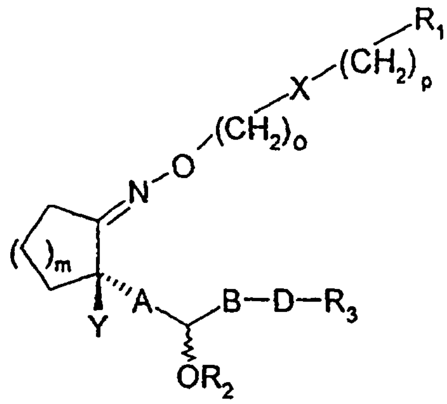

(I)

wonn

- $R_1$ H, $CF_3$, $CH_2OH$, $COOR_4$, $CONR_5R_6$, und
- $R_2$ H oder einen organischen Säurerest mit 1-15 C-Atomen darstellt,
- $R_3$ H, gegebenenfalls einfach oder mehrfach substituiertes $C_1$-$C_{14}$-Alkyl, $C_3$-$C_{10}$-Cycloalkyl, einen gegebenenfalls unabhängig voneinander einfach oder mehrfach durch Halogen, Phenyl, $C_1$-$C_4$-Alkyl, $C_1$-$C_4$-Alkoxy, Fluormethyl, Chlomnethyl. Trifluormethyl, Carbonyl, Carboxyl oder Hydroxyl substituierten $C_6$-$C_{10}$-Arylrest oder einen 5-6 gliedrigen aromatischen heterocyclischen Ring mit wenigstens 1 Heteroatom symbolisiert,
- $R_4$ Wasserstoff, $C_1$-$C_{10}$-Alkyl, $C_3$-$C_{10}$-Cycloalkyl, einen gegebenenfalls durch 1-3 Halogen, Phenyl, $C_1$-$C_4$-Alkyl, $C_1$-$C_4$-Alkoxy, Fluormethyl, Chlormethyl, Trifluormethyl, Carboxyl oder Hydroxyl substituierten $C_6$-$C_{10}$-Arylrest, $CH_2$-CO-($C_6$-$C_{10}$) Aryl oder einen 5-6-gliedrigen Ring mit wenigstens 1 Heteroatom bedeutet,
- A eine trans, trans-CH=CH-CH=CH, eine $-CH_2CH_2$-CH=CH- oder eine Tetramethylengruppe,
- B eine $C_1$-$C_{10}$- gerad- oder verzweigtkettige Alkylengruppe, die gegebenenfalls durch Fluor substituiert sein kann oder die Gruppe

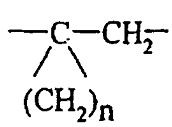

oder

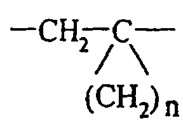

symbolisiert,

- D eine Direktbindung, Sauerstoff, Schwefel, -C≡C-, $-CH=CR_7$, oder gemeinsam mit
- B auch eine Direktbindung bedeuten kann,
- $R_5$ und $R_6$ gleich oder verschieden sind und H oder gegebenenfalls durch Hydroxylgruppen substituiertes $C_1$-$C_4$-Alkyl oder $R_6$ H und $R_5$ $C_1$-$C_{15}$-Alkanoyl oder $R_8SO_2$- darstellen,
- $R_7$ H, $C_1$-$C_5$-Alkyl, Chlor, Brom bedeutet,
- $R_8$ die gleiche Bedeutung wie $R_3$ besitzt,
- m 1-3 bedeutet
- o 0-5 bedeutet,
- p 0-5
- X eine Direktbindung, Sauerstoff, Schwefel, einen Aromaten oder Heteroaromaten,
- Y ein gegebenenfalls einfach oder mehrfach substituiertes $C_1$-$C_8$-Alkyl, $C_3$-$C_{10}$-Cydoalkyl, gegebenenfalls substituiert durch Aryl,
- n 2-5 ist

und, wenn $R_4$ Wasserstoff bedeutet, deren Salze mit physiologisch verträglichen Basen und deren Cyclodextrinclathrate.

**2.** Pharmazeutische Präparate **gekennzeichnet durch** einen Gehalt an Leukotrien-$B_4$-Derivaten der allgemeinen Formel I gemäß Patentanspruch 1.

**3.** Verfahren zur Herstellung von Leukotrien-$B_4$-Derivaten der allgemeinen Formel I, gemäß Patentanspruch 1, **dadurch gekennzeichnet, daß** man ein Keton der Formel II

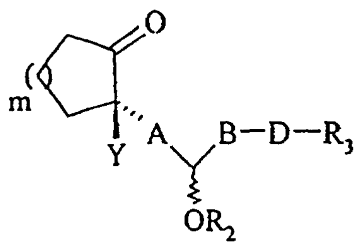

(II)

worin A, B, D, m, $R_2$, $R_3$ und Y die oben angegebene Bedeutung haben, gegebenenfalls nach Schutz freier Hydroxylgruppen in $R_2$ mit einem Hydroxylamin oder Hydroxylammoniumsalz umsetzt und anschließend mit einem Alkylierungsreagenz der allgemeinen Formel III,

$$E\text{-}(CH_2)_o\text{-}X\text{-}(CH_2)_p\text{-}R_1 \qquad \text{(III)}$$

wobei E ein Halogenid oder Sulfonat darstellt und o, X, p, $R_1$ die oben angegebenen Bedeutungen aufweisen, in Gegenwart einer Base umsetzt und gegebenenfalls anschließend in beliebiger Reihenfolge Isomere trennt, geschützte Hydroxylgruppen freisetzt und/ oder eine freie Hydroxylgruppe verethert und/ oder eine Carboxylgruppe reduziert und/ oder eine Carboxylgruppe verestert und/ oder eine Carboxylgruppe in ein Amid überführt oder eine Carboxylgruppe mit einer physiologisch verträglichen Base in ein Salz überführt.

**Claims**

**1.** Leukotriene $B_4$ derivatives of the general formula I

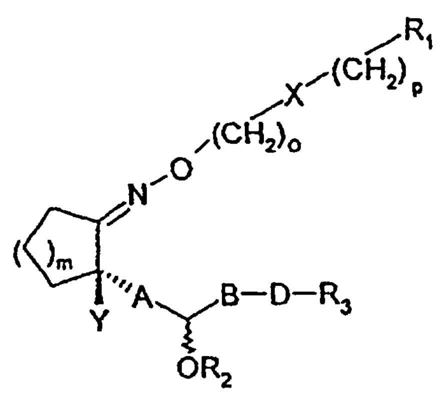

(I)

in which

- $R_1$ is H, $CF_3$, $CH_2OH$, $COOR_4$, $CONR_5R_6$, and
- $R_2$ is H or an organic acid residue with 1-15 C atoms,
- $R_3$ is H, optionally mono- or multisubstituted $C_1$-$C_{14}$-alkyl, $C_3$-$C_{10}$-cycloalkyl, a $C_6$-$C_{10}$-aryl radical which is optionally substituted independently of one another one or more times by

halogen, phenyl, $C_1$-$C_4$-alkyl, $C_1$-$C_4$-alkoxy, fluoromethyl, chloromethyl, trifluoromethyl, carbonyl, carboxyl or hydroxyl,

or

a 5-6-membered aromatic heterocyclic ring with at least 1 heteroatom,

$R_4$ is hydrogen, $C_1$-$C_{10}$-alkyl, $C_3$-$C_{10}$-cycloalkyl, a $C_6$-$C_{10}$-aryl radical which is optionally substituted by 1-3 halogen, phenyl, $C_1$-$C_4$-alkyl, $C_1$-$C_4$-alkoxy, fluoromethyl, chloromethyl, trifluoromethyl, carboxyl or hydroxyl, or $CH_2$-CO-($C_6$-$C_{10}$)aryl

or

a 5-6-membered ring with at least 1 heteroatom,

A is a trans, trans-CH=CH-CH=CH, a -$CH_2CH_2$-CH=CH- or a tetramethylene group,

B is a $C_1$-$C_{10}$ straight- or branched-chain alkylene group which may optionally be substituted by fluorine, or the group

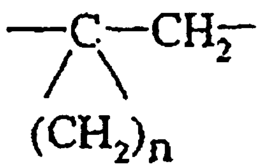

or

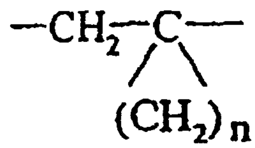

D may be a direct linkage, oxygen, sulphur, -C≡C-, -CH=$CR_7$, or together with

B also a direct linkage,

$R_5$ and $R_6$ are identical or different and are H or $C_1$-$C_4$-alkyl which is optionally substituted by hydroxyl groups, or $R_6$ is H and $R_5$ is $C_1$-$C_{15}$-alkanoyl or $R_8SO_2$-,

$R_7$ is H, $C_1$-$C_5$-alkyl, chlorine, bromine,

$R_8$ has the same meaning as $R_3$,

m is 1-3,

o is 0-5,

p is 0-5,

X is a direct linkage, oxygen, sulphur, an aromatic or heteroaromatic system,

Y is an optionally mono- or multisubstituted $C_1$-$C_8$-alkyl, $C_3$-$C_{10}$-cycloalkyl, optionally substituted by aryl,

n is 2-5,

and, when $R_4$ is hydrogen, the salts thereof with physiologically tolerated bases and the cyclodextrin clathrates thereof.

2. Pharmaceutical products **characterized by** a content of leukotriene $B_4$ derivatives of the general formula I according to Claim 1.

3. Process for the preparation of the leukotriene $B_4$ derivatives of the general formula I according to Claim 1, **characterized in that** a ketone of the formula II

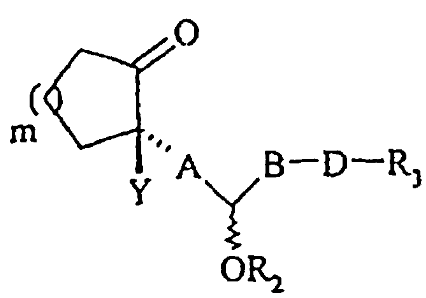

(II)

in which A, B, D, m, $R_2$, $R_3$ and Y have the meanings stated above, where appropriate after protection of free hydroxyl groups in $R_2$, is reacted with a hydroxylamine or hydroxylammonium salt, and subsequently reacted with an alkylating reagent of the general formula III

$$E\text{-}(CH_2)_o\text{-}X\text{-}(CH_2)_p\text{-}R_1 \qquad (III)$$

where E is a halide or sulphonate, and o, X, p, $R_1$ have the meanings stated above, in the presence of a base, and, where appropriate, subsequently in any sequence isomers are separated, protected hydroxyl groups are liberated and/or a free hydroxyl group is etherified and/or a carboxyl group is reduced and/or a carboxyl group is esterified and/or a carboxyl group is converted into an amide, or a carboxyl group is converted with a physiologically tolerated base into a salt.

## Revendications

**1.** Dérivés de leucotriène $B_4$ de formule générale I,

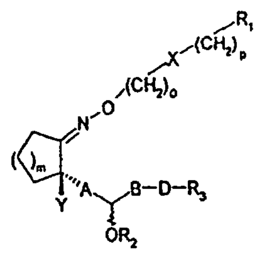

(I)

dans laquelle

| | |
|---|---|
| $R_1$ | représente H, $CF_3$, $CH_2OH$, $COOR_4$, $CONR_5R_6$, et |
| $R_2$ | représente H ou un radical acide organique ayant de 1 à 15 atomes de carbone, |
| $R_3$ | représente H, un alkyle en $C_1$-$C_{14}$ éventuellement mono- ou polysubstitué, un cycloalkyle en $C_3$-$C_{10}$, un radical aryle en $C_6$-$C_{10}$ éventuellement mono- ou polysubstitué indépendamment par un halogène, un phényle, un alkyle en $C_1$-$C_4$, un alcoxy en $C_1$-$C_4$, un fluorométhyle, un chlorométhyle, un trifluorométhyle, un carbonyle, un carboxy ou un hydroxy, ou un noyau hétérocyclique aromatique à 5-6 chaînons ayant au moins 1 hétéroatome, |
| $R_4$ | représente un hydrogène, un alkyle en $C_1$-$C_{10}$, un cycloalkyle en $C_3$-$C_{10}$, un radical aryle en $C_6$-$C_{10}$ éventuellement mono- à trisubstitué par un halogène, un phényle, un alkyle en $C_1$-$C_4$, un alcoxy en $C_1$-$C_4$, un fluorométhyle, un chlorométhyle, un trifluorométhyle, un carboxy ou un hydroxy, un $CH_2$-CO-($C_6$-$C_{10}$)-aryle ou un noyau à 5-6 chaînons ayant au moins 1 hétéroatome, |
| A | représente un groupe trans, trans-CH=CH-CH=CH, -$CH_2$-$CH_2$-CH=CH- ou tétraméthylène, |
| B | représente un groupe alkylène en $C_1$-$C_{10}$ linéaire ou ramifié qui peut être éventuellement substitué par un fluor, ou le groupe |

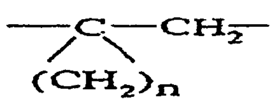 ou 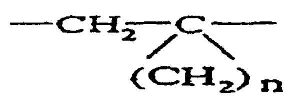

| | |
|---|---|
| D | représente une liaison directe, un hydrogène, un soufre, un -C=C-, un -CH=$CR_7$, ou conjointement avec |
| B | peut également représenter une liaison directe, |
| $R_5$ et $R_6$ | sont identiques ou différents et représentent H ou un alkyle en $C_1$-$C_4$ éventuellement substitué par des groupes hydroxy, ou $R_6$ représente H et $R_5$ représente un alcanoyle en $C_1$-$C_{15}$ ou $R_8SO_2$-, |
| $R_7$ | représente H, un alkyle en $C_1$-$C_5$, un chlore ou un brome, |
| $R_8$ | présente la même signification que $R_3$, |
| m | vaut de 1 à 3, |

o vaut de 0 à 5,
p vaut de 0 à 5,
X représente une liaison directe, un oxygène, un soufre, une structure aromatique ou une structure hétéroaromatique,
Y représente un alkyle en $C_1$-$C_8$ éventuellement mono- ou polysubstitué ou un cycloalkyle en $C_3$-$C_{10}$ éventuellement substitué par un aryle,
n vaut de 2 à 5,
et, si $R_4$ représente un hydrogène, leurs sels avec des bases physiologiquement compatibles et leurs clathrates de cyclodextrine.

**2.** Préparations pharmaceutiques **caractérisées par** une teneur en dérivés de leucotriène $B_4$ de formule générale I selon la revendication 1.

**3.** Procédé de préparation de dérivés de leucotriène $B_4$ de formule générale I selon la revendication 1, **caractérisé en ce qu'**une cétone de formule II

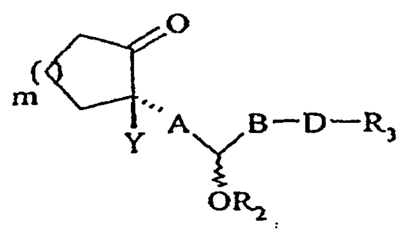
(II)

dans laquelle A, B, D, m, $R_2$, $R_3$ et Y ont la signification indiquée ci-dessus, éventuellement après protection des groupes hydroxy libres de $R_2$, est mise à réagir avec une hydroxylamine ou un sel d'hydroxylammonium et ensuite mise à réagir avec un réactif d'alkylation de formule générale III

$$E\text{-}(CH_2)_o\text{-}X\text{-}(CH_2)_p\text{-}R_1 \quad \text{(III)}$$

dans laquelle E représente un halogénure ou un sulfonate et o, X, p et $R_1$ présentent les significations indiquées ci-dessus, en présence d'une base, et ensuite éventuellement, dans un ordre quelconque, les isomères sont séparés, les groupes hydroxy protégés sont libérés et/ou un groupe hydroxy libre est éthérifié et/ou un groupe carboxy est réduit et/ou un groupe carboxy est estérifié et/ou un groupe carboxy est transformé en amide ou un groupe carboxy est transformé en sel avec une base physiologiquement compatible.